# EUROPEAN PATENT SPECIFICATION

(11) **EP 2 996 677 B1**
(45) Date of publication and mention of the grant of the patent: **20.11.2019**
(21) Application number: 14801516.7
(22) Date of filing: 16.05.2014
(51) Int. Cl.: A61K 41/00, A61K 9/14, A61K 47/30, A61K 49/12, A61K 47/59, A61K 47/69

(54) **PHOTOSENSITIZER PARTICLES FOR MEDICAL IMAGING AND/OR PHOTODYNAMIC THERAPY**
FOTOSENSIBILISATORTEILCHEN FÜR DIE MEDIZINISCHE BILDGEBUNG UND/ODER FOTODYNAMISCHE THERAPIE
PARTICULES DE PHOTOSENSIBILISATEUR DESTINÉES À L'IMAGERIE MÉDICALE ET/OU À LA THÉRAPIE PHOTODYNAMIQUE

(30) Priority: 18.05.2013 US 201361824992 P
(43) Date of publication of application: 23.03.2016
(73) Proprietor: National Chung-Hsing University, Taichung City (TW)
(72) Inventor: LAI, Ping-Shan, Taichung City 402 (TW); HSU, Chia-Yen, 325 Longtan Township Taoyuan County (TW)
(74) Representative: Lang, Christian
(86) International application number: PCT/US2014/038488
(87) International publication number: WO 2014/189796

(56) References cited:
- WO-A1-2010/123918
- WO-A1-2010/133700
- WO-A1-2010/143942
- WO-A1-2011/038043
- WO-A1-2013/020204
- WO-A2-2013/012628
- US-A1- 2004 047 913
- US-A1- 2005 079 131
- US-A1- 2007 258 889
- US-A1- 2009 304 803
- US-A1- 2011 125 079
- US-A1- 2012 014 874
- US-A1- 2012 244 078

## Description

### BACKGROUND OF THE INVENTION

### 1. FIELD OF THE INVENTION

The present disclosure relates to photosensitizer particles for medical imaging and/or photodynamic therapy (PDT) as defined in the claims. More particularly, the disclosed invention relates to photosensitizer particle-based contrast agents and PDT agents, and methods for making and using such agents.

### 2. DESCRIPTION OF RELATED ART

Medical imaging is the technique and process used to create images of the body part(s) of animals, including human, for diagnostic, therapeutic and/or scientific purposes. Various techniques have been used in the art to image different tissues and organs for a wide range of diagnostic and/or therapeutic purposes.

When performing medical imaging, contrast-enhancing agents (or contrast agents) are often used to contrast between different tissues, in order to improve the visibility of tissues of interest. Generally, the contrast agents possess a property that can be detected by a particular detection device, and when introduced into the body of a subject, the presence of the contrast agent at the site of interest allows an image of the site to be created, thus allowing the medical practitioner to assess the site.

Diagnostic or medical sonography (ultrasonography) is an ultrasound-based imaging technique used for visualizing subcutaneous tissues/organ in a subject. The concept of ultrasonography differs from other medical imaging modalities in the fact that it is operated by the transmission and receipt of sound waves. High frequency broadband sound waves are sent into the tissue and the sound waves are then reflected by the tissues. As could be appreciated, sound waves reflected by different tissues would result in different pattern depending on the composition and structures of these tissues to produce 2-dimentional or 3-dimentional images.

Contrast-enhanced ultrasonography (CEUS) is the application of ultrasound contrast agent to traditional medical sonography. Commercially available ultrasound contrast agents are highly echogenic materials such as gas-filled microbubbles. Echogenicity is the ability of an object to reflect the ultrasound waves, and the presence of the microbubble at the target site would enhance the reflection of the ultrasound waves to produce a unique sonogram with increased contrast due to the high echogenicity difference between the microbubble and the soft tissues surrounding the target site. Generally, contrast-enhancing microbubbles are injected intravenously into the systemic circulation in a small bolus, and when the microbubbles in the blood flow past the target site, the microbubbles reflect a unique echo that stands in stark contrast to the surrounding tissue. However, microbubbles have low circulation residence times because they either get taken up by immune system cells or organs such as liver and spleen.

Magnetic resonance imaging (MRI) is a commonly used medical imaging technique to visualize detailed internal structures of a subject. For MRI examination, the subject is exposed to a powerful magnetic field and a radiofrequency is applied, thereby causing some atoms in the subject's body to spin and then relax after the pulse stops. This relaxation emits energy which is detected by the MRI scanner and is digitally converted into an image. Although MRI has good contrast between different soft tissues of the body, and hence an MRI contrast agent is not a requisite for most MRI examinations; MRI contrast agents are often administered to the subject, in particular when fine MRI examination is to be performed. These MRI contrast agents, upon delivery to the target site, alter the relaxation times of atoms within the subject's body thereby enhancing the contrast.

Based on their magnetic properties, MRI contrast agents can be classified into paramagnetic and superparamagnetic MRI contrast agents. Paramagnetic MRI agents comprise a paramagnetic ion (such as, gadolinium and manganese) which is often in the form of chelates. Most clinically approved paramagnetic MRI contrast agents are small molecule based; however, small molecule contrast agents may suffer from certain disadvantages such as short blood circulation time, lower sensitivity, high viscosity, and high osmolality. These compounds generally have been associated with renal complications in some patient populations. Also, these small molecule agents clear from the body rapidly, making it difficult to target these agents to the site of interest. Superparamagnetic MRI contrast agents are photosensitizer particles of iron oxide or iron platinum. However, there is only one oral iron oxide contrast agent (LUMIREM®, also known as GASTROMARK™) that is approved for clinical use in human.

While there are various contrast-enhancing agents available for diagnostic and therapeutic uses, the present invention has recognized that there still exists a need in the art for contrast-enhancing agents that can target and deliver contrast-enhancing agents and/or bioactive agents to organs, tissues, and cells of animals.

Photodynamic therapy (PDT) has been used for treating various diseases including localized solid tumors, ophthalmological diseases, and skin conditions. Oxygen is an essential component of PDT. In PDT, photosensitizing agents are administered to cells, and then light of suitable wavelength and intensity is used to activate the photosensitizing agents. The activated photosensitizing agents react with oxygen to produced reactive oxygen species (Type I PDT) or singlet oxygen (Type II PDT), which in turn trigger the destruction of diseased cells. Therefore, the presence of oxygen at the treatment site is crucial to the efficacy of the therapy. However, in solid tumors, the growth rate of tumor cells is often greater than the rate of blood vessel formation, thereby resulting in a condition referred to as hypoxia in which tumor cells become deficient in oxygen. The exposure of tumor cells to a hypoxic environment compromises PDT in its ability to kill malignant cells. WO 2011/038043 A1 discloses an injectable microbubble contrast agent composition comprising a gas core encapsulated by a monolayer shell, said shell comprising a first surfactant, a second surfactant having a higher water solubility than said first surfactant, an anchor molecule attached to said shell at the gas-shell interface and an optically-active probe, said probe being detectable by fluorescence, near-infrared, bioluminescence, or other optical imaging methods. WO 2010/143942 A1 discloses a photosensitizer covalently bound to a nanoparticle precursor, which can be e.g. PLA or PLGA, and from which a nanoparticle is formed. The photosensitizer is covalently bound throughout at least a part of the nanoparticle and incorporated therein as a mixture of monomeric and aggregated molecules. The nanoparticle can be used for killing cancer cells by PDT.

Hence, there is an urgent need for a method of treatment of hypoxic malignant tumors that is capable of delivering an oxygenating agent directly to the tumor site to ensure a more effective photodynamic therapy.

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later. The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

In one aspect, the present disclosure is directed to photosensitizer particles suitable for use in ultrasonography, magnetic resonance imaging (MRI), or photodynamic therapy.

According to one embodiment of the present disclosure, the photosensitizer particle comprises a core and a shell. The core is filled with gas, liquid or a mixture of gas and liquid. The shell consists essentially of a plurality of photosensitizer conjugates, and each of the photosensitizer conjugates consists of a photosensitizer and one or more biodegradable polymer covalently bound to the photosensitizer.

In certain embodiments of the present disclosure, the core is filled with gas or a mixture of gas and liquid, and the gas may contain air, oxygen, and/or fluorocarbons.

According to various embodiments, the photosensitizer is porphyrin, chlorin, phthalocyanine, bacteriochlorin, methylene blue, or a derivative thereof. In certain embodiments, the biodegradable polymer is polylactic acid (PLA), polycaprolactone (PCL), or polyglycolic acid (PGA).

Each of the photosensitizer conjugate has 1 to 4 biodegradable polymers covalently bound to the photosensitizer. Optionally, the shell has an aggregation number of about 2,000-15,000.

The core of the photosensitizer particle has a radius of about 10-100 nm on average, whereas the shell of the photosensitizer particle has a thickness of about 10-100 nm, according to various embodiments of the present disclosure. Also, the photosensitizer particle has a polydispersity index of about 0,05-0.3.

According to optional embodiment of the present disclosure, the photosensitizer is *meta*-tetra-3-hydroxymethyl phenyl chlorin (m-THPMPC) and the biodegradable polymer is polylactic acid.

In certain optional embodiments, the core is filled with gas and the photosensitizer particle is prepared by the method as follows. First, the photosensitizer conjugates are dissolved in an organic solvent in a ratio of 1:10 to 1:100 (m/v) to produce a conjugate solution. Then, the conjugate solution is drop-wisely added into deionized water with stirring while gas is pumped into the mixture. The organic solvent is acetone or tetrahydrofuran, and the volume ratio of the organic solvent to the deionized water is 1:1 to 1:20. The pumping is continued until at least 30 minutes after the depletion of the conjugate solution.

In some other embodiments, the core is filled with gas and the photosensitizer particle is prepared by the method as follows. First, the photosensitizer conjugates are dissolved in dichloromethane in a ratio of 1:1 to 100:1 (m/v) to produce a conjugate solution. Then, a first polyvinyl alcohol (PVA) aqueous solution is added into the conjugate solution with sonication to produce a water-in-oil emulsion. Next, the water-in-oil emulsion is added into a second PVA solution with sonication to produce a water-in-oil-in-water emulsion. Thereafter, the dichloromethane is removed from the water-in-oil-in-water emulsion, and then the PVA is removed to produce the photosensitizer particle. In certain embodiments of the present disclosure, the concentration of the first or second PVA solution is 0.1 to 10% (w/v), the volume ratio of the dichloromethane to the first PVA aqueous solution is 1:1 to 100:1, and the volume ratio of the dichloromethane to the second PVA aqueous solution is 1:1 to 1:10. According to various embodiments, the PVA is removed by centrifugation.

According to some embodiments of the present disclosure, the photosensitizer particle is suitable for use in MRI, and the photosensitizer of at least one of the plurality of photosensitizer conjugates comprises a magnetic contrast-enhancing material. The core is filled with liquid, gas, or a combination of the two.

In certain embodiments of the present disclosure, the magnetic contrast-enhancing material is a paramagnetic ion which is chelated to the photosensitizer. Examples of such paramagnetic ion include, but are not limited to, manganese (II), manganese (III), gadolinium (III), iron (III), iron (II), chromium (III), cobalt (II), nickel (II), copper (II), neodymium (III), samarium (III), ytterbium (III), vanadium (II), terbium (III), dysprosium (III), holmium (III) and erbium (III).

According to some embodiments of the present disclosure, the liquid filled in the core of the photosensitizer particle may optionally contain one or more oxygen carriers, such as hemoglobin-based oxygen carriers or perfluorocarbon-based oxygen carriers.

In yet another aspect, the present disclosure is directed to a method for imaging a body part of a subject.

According to various embodiments of the present disclosure, the method comprises the steps as follows. In an administration step, an imaging composition comprising the photosensitizer particle contrast agent according to any of the above aspects/embodiments of the present disclosure is administered to the subject. In an imaging step, the body part of interest is imaged by either sonographic imaging or magnetic resonance imaging.

For example, photosensitizer particles having cores filled with gas or a mixture of gas and liquid are suitable for use in sonographic imaging, whereas photosensitizer particles comprising a magnetic contrast-enhancing material are suitable for use in MRI imaging. Additionally, photosensitizer particles that have cores filled with gas or a mixture of gas and liquid and comprise a magnetic contrast-enhancing material may exhibit dual modalities, and hence are suitable for use in both sonographic and MRI imaging.

In still another aspect of the present disclosure, provided are the photosensitizer particles for use in a method for treating a subject suffering from a disease.

According to certain embodiments of the present disclosure, the method comprises the steps as follows. First, a pharmaceutical composition comprising an effective amount of photosensitizer particles according to the first aspects of the present disclosure (and associated embodiments) is administered to a disease site of the subject. Thereafter, the disease site of the subject is irradiated with a light sufficient to activate the photosensitizer. Specifically, the photosensitizer particles are filled with gas or a combination of gas and liquid in the core.

According to various embodiment of the present disclosure, the disease may be non-small-cell lung cancer, prostate cancer, esophageal cancer, skin cancer, breast cancer, bladder cancer, pancreatic cancer, Karposi's sarcoma, retinoblastoma, age-related macular degeneration, psoriasis, arthritis, or photoangioplasty of peripheral arterial disease.

In optional embodiments, the light has a wavelength in the range of about 550 to 750 nm; preferably, about 650 to 700 nm. Further, the light intensity of the irradiation is about 20 to 200 J/cm².

In still another aspect, the present disclosure is directed to use of a photosensitizer particle for the manufacture of a medicament for use in the photodynamic therapy.

According to certain embodiments of the present disclosure, the photosensitizer particle comprises a core and a shell. The core is filled with gas, liquid, or a mixture of gas and liquid. The shell consists essentially of a plurality of photosensitizer conjugates, and each of the photosensitizer conjugates consists of a photosensitizer and one or more biodegradable polymer covalently bound to the photosensitizer.

According to various embodiments, the photosensitizer is porphyrin, chlorin, phthalocyanine, bacteriochlorin, methylene blue, or a derivative thereof. In certain embodiments, the biodegradable polymer is polylactic acid (PLA), polycaprolactone (PCL), or polyglycolic acid (PGA). According to optional embodiment of the present disclosure, the photosensitizer is *meta*-tatra-3-hydroxymethyl phenyl chlorin (m-THPMPC) and the biodegradable polymer is polylactic acid. Each of the photosensitizer conjugate has 1 to 4 biodegradable polymers covalently bound to the photosensitizer.

According to various embodiment of the present disclosure, the photodynamic therapy is used to treat non-small-cell lung cancer, prostate cancer, esophageal cancer, skin cancer, breast cancer, bladder cancer, pancreatic cancer, Karposi's sarcoma, or retinoblastoma.

According to some embodiments of the present disclosure, the liquid filled in the core of the photosensitizer particle may optionally contain one or more oxygen carriers, such as hemoglobin-based oxygen carriers or perfluorocarbon-based oxygen carriers.

Many of the attendant features and advantages of the present disclosure will becomes better understood with reference to the following detailed description considered in connection with the accompanying drawings.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The present description will be better understood from the following detailed description read in light of the accompanying drawings, where:
Figure 1A is a schematic diagram illustrating the general structure of the photosensitizer particle 100 according to certain embodiments of the present invention;
Figure 1B is the fluorescence spectra of PPLA (Example 1.1) and CPLA (Example 1.2) conjugates according to the present disclosure;
Figure 2 is a schematic diagram illustrating the photosensitizer particle 200 for use in MRI according to another embodiment of the present invention;
Figure 3 is a representative TEM image of gas-loaded photosensitizer particles for use in ultrasonic imaging according to yet another embodiment of the present invention;
Figure 4 is a representative TEM image of photosensitizer particles according to still another embodiment of the present invention;
Figure 5A, Figure 5B, and Figure 5C are ultrasonic images of the experimental and control groups were according to one working example of the present invention;
Figure 6 is a bar graph illustrating the fluorescence intensity measured in one working example of the present invention;
Figure 7 present microscopic photographs illustrating cell morphology before and after photodynamic therapy according to one working example of the present invention;
Figure 8 present fluorescent photographs illustrating the production of reactive oxygen species resulted from photodynamic therapy according to one working example of the present invention;
Figure 9A and Figure 9B are line graphs illustrating cell viability according to one working example of the present invention;
Figure 10A and Figure 10B are line graphs illustrating tumor-inhibitory activity and change of body weight according to one working example of the present invention;
Figure 11 is a T1-weighted MRI scan image of samples containing different concentrations of Mn-CPCL photosensitizer particles according to another working example of the present invention;
Figure 12 is a line graph illustrating the production of singlet oxygen mediated by the photosensitizer particles according to one working example of the present invention;
Figure 13A and Figure 13B are line graphs illustrating cell viability according to another working example of the present invention;
Figure 14 is a line graph illustrating the production of singlet oxygen mediated by the photosensitizer particles according to yet another working example of the present invention;
Figure 15A and Figure 15B are line graphs illustrating cell viability according to still another working example of the present invention;
Figure 16A and Figure 16B are line graphs illustrating tumor-inhibitory activity and change of body weight according to still another working example of the present invention;
Figure 17 is a line graph illustrating the hemolytic activity of the present photosensitizer particles according to one working example of the present invention; and
Figure 18 provides photographs illustrating the *in vivo* photohypersensitivity of mice skin to the present photosensitizer particles according to one working example of the present invention.

In accordance with common practice, the various described features/elements are not drawn to scale but instead are drawn to best illustrate specific features/elements relevant to the present invention. Also, like reference numerals and designations in the various drawings are used to indicate like elements/parts.

### DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

For convenience, certain terms employed in the specification, examples and appended claims are collected here. Unless defined otherwise, all technical and scientific terms used herein have the same meaning as commonly understood by one of the ordinary skill in the art to which this invention belongs.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." At the very least, each numerical parameter should at least be construed in light of the number of reported significant digits and by applying ordinary rounding techniques.

Unless otherwise defined herein, scientific and technical terminologies employed in the present disclosure shall have the meanings that are commonly understood and used by one of ordinary skill in the art. Unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

The term "contrast-enhancing materials," as used herein, refers to materials capable of being monitored, after being administered to a subject, by methods for monitoring and detecting such materials, for example by MRI or sonographic imaging. The term "contrast agent" is used herein to refer to an agent comprising the contrast enhancing materials, and is used to highlight a target site so that organs, tissues and/or cells of interest are more visible by the imaging procedure. In particular, the contrast agent is the photosensitizer particle according to various embodiments of the present disclosure. Also, as used herein, the term "imaging composition" refers to a composition for enhancing contrast in an MRI or sonographic imaging procedure. For example, the imaging composition may comprise the photosensitizer particle contrast agent according to various embodiments of the present disclosure, as well as a pharmaceutically acceptable carrier for administration to a subject.

The term "biodegradable" generally refers to a polymer which degrades into component subunits in a biological environment by e.g., hydrolysis or enzymatic reactions. For example, one type of biodegradation may involve cleavage of bonds in the polymer backbone, which results in monomers and oligomers. In contrast, another type of biodegradation may involve cleavage of a bond internal to a side chain or that connects a side chain to the polymer backbone, and in this case, the chemical moiety attached as a side chain to the polymer backbone may be released by biodegradation.

As used herein, the term "aggregation number" means the number of polymer molecules which together form a particle (or aggregate).

Here, the term "polydispersity" is an indicator of the width of the particle size distribution of photosensitizer particles. According to the present disclosure, the polydispersity index (PDI) of photosensitizer particles is measured by dynamic light scattering (DLS) and defined as: PDI = (σ/d)², where σ is the standard deviation of the sample, and d is the mean diameter thereof. In this sense, PDI values are in the range from 0 to 1. A sample is monodisperse if the PDI is less than 0.2, it is medium disperse if the PDI is in the range of 0.2 to 0.3, and it is polydisperse if the PDI is more than 0.3. For synthetic polymers, such as the present photosensitizer conjugates, polydispersity index (PDI) is used to characterize the dispersions of distributions of molar masses. PDI of a polymer mixture is often measured by gel permeation chromatography (GPC). In this context, the PDI from polymerization is denoted as: PDI = (M_{w}/Mₙ), where M_{w} is weight-average molecular weight while Mₙ is number-average molecular weight. PDI value of a polymer mixture is equal to or greater than 1, and for a uniform polymer, PDI = 1.

The terms "treatment" and "treating" are used herein to include curative or palliative treatment that results in a desired pharmaceutical and/or physiological effect. Preferably, the effect is therapeutic in terms of partially or completely curing cancer. In particular, the term "treating" to application or administration of the physical and/or chemical intervention to a subject, who has a medical condition, a symptom of the condition, a disease or disorder secondary to the condition, or a predisposition toward the condition, with the purpose to partially or completely alleviate, ameliorate, relieve, delay onset of, inhibit progression of, reduce severity of, and/or reduce incidence of one or more symptoms or features of a particular disease, disorder, and/or condition. Treatment may be administered to a subject who exhibits only early signs of a disease, disorder, and/or condition for the purpose of decreasing the risk of developing pathology associated with the disease, disorder, and/or condition. According to certain embodiments of the present disclosure, said disease, disorder, or condition is cancer. Treatment is generally "effective" if one or more symptoms or clinical markers are reduced as that term is defined herein.

The term "effective amount" as used herein refers to the quantity of a component which is sufficient to yield a desired response. The specific effective amount will vary with such factors as the particular condition being treated, the physical condition of the subject (e.g., the subject's body mass, age, or gender), the type of mammal or animal being treated, the duration of the treatment, the nature of concurrent therapy (if any), and the specific formulations employed. An effective amount is also one in which any toxic or detrimental effects of the compound or composition are outweighed by the therapeutically beneficial effects.

The terms "application" and "administration" are used interchangeably herein to refer means providing the photosensitizer particles, imaging compositions, or a pharmaceutical composition of the present invention to a subject for medical imaging and/or treating purposes. As could be understood, the terms "application" and "administration" include all routes of administration known in the art, including but not limited to, oral, topical, transdermal, parenteral, subcutaneous, intranasal, mucosal, intramuscular, intraperitoneal, intravitreal and intravenous routes, including both local and systemic applications. In certain working example, the present pharmaceutical composition is intravenously injected into the subject.

The term "excipient" as used herein means any inert substance (such as a powder or liquid) that forms a vehicle/carrier for the photosensitizer particles of the present disclosure. The excipient is generally safe, non-toxic, and in a broad sense, may also include any known substance in the pharmaceutical industry useful for preparing imaging and/or pharmaceutical compositions such as, fillers, diluents, agglutinants, binders, lubricating agents, glidants, stabilizer, colorants, wetting agents, disintegrants, and etc.

As used herein, a "pharmaceutically acceptable" component is one that is suitable for use with humans and/or animals without undue adverse side effects (such as toxicity, irritation, and allergic response) commensurate with a reasonable benefit/risk ratio. Also, each excipient must be "acceptable" in the sense of being compatible with the other ingredients of the pharmaceutical formulation. The carrier can be in the form of a solid, semi-solid, or liquid diluent, cream or a capsule.

The term "subject" refers to a mammal including the human species that is treatable with the photosensitizer particles and/or methods of the present invention. The term "subject" is intended to refer to both the male and female gender unless one gender is specifically indicated.

The present invention is directed to photosensitizer particles suitable for uses as contrast agents and/or therapeutic agents as defined in the claims. Alternatively, the photosensitizer particles can be used to improve the imaging/contrasting efficiency over conventional imaging techniques. Still alternatively, the photosensitizer particles can be used to improve the therapeutic efficiency over conventional therapeutic techniques. For imaging application, the photosensitizer particles may comprise a contrast-enhancing material such as paramagnetic ions or gas. Such photosensitizer particles or imaging compositions comprising them, when administered to a subject, improve the detection limit or detection capability of a method, technique, or apparatus for medical imaging (e.g. MRI and ultrasound). Accordingly, these imaging methods, techniques, and/or apparatus also fall within the scope of the present disclosure. For therapeutic application, the photosensitizer particles comprising gas, liquid or a mixture of the two can be used as the active agents in PDT. Hence, the pharmaceutical compositions comprising such photosensitizer particles, as well as treating methods using the same, also fall within the scope of the present disclosure.

Generally, the photosensitizer particle has a shell/core structure and is formed from self-assembly of a plurality of photosensitizer conjugates. Depending on the manufacturing process, the core may be filled with liquid, gas, or a combination of both.

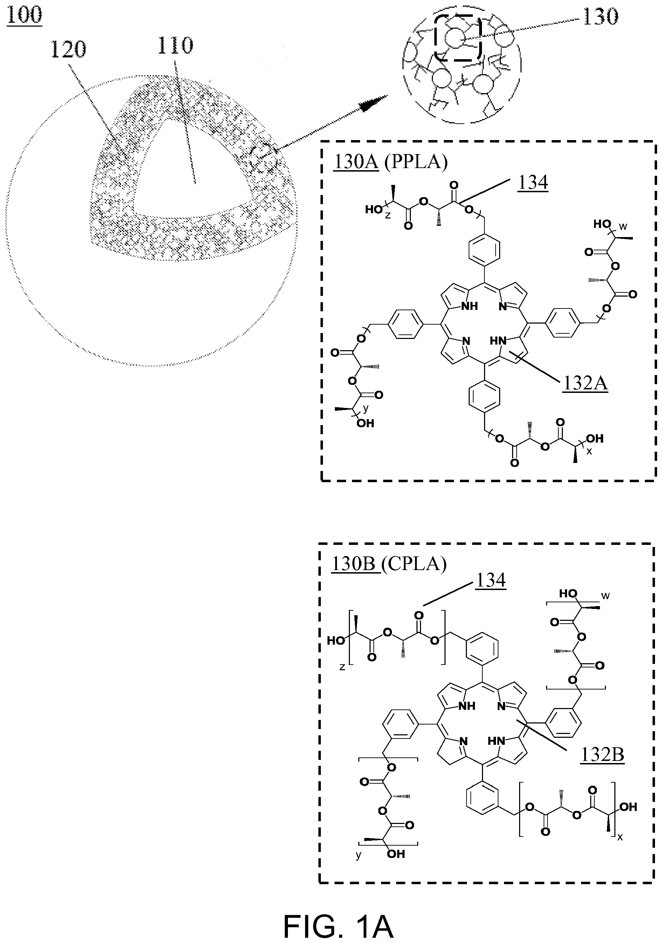

Figure 1A is a schematic diagram illustrating the general structure of the photosensitizer particle 100 according to the present invention. The photosensitizer particle 100 is a hollow structure which comprises a core 110 and a shell 120 encasing the core 110. The shell 120 consists essentially of a plurality of photosensitizer conjugates 130. Generally, each photosensitizer conjugate 130 consists of a photosensitizer and one or more biodegradable polymer covalently bound to the photosensitizer. According to various aspect/embodiments of the present disclosure, the photosensitizer particle 100 could be used as a contrast agent and/or a PDT agent.

Examples of the photosensitizer include porphyrin, chlorin, phthalocyanine, bacteriochlorin, methylene blue, and derivative thereof. Examples of biodegradable polymer include, but are not limited to, polylactic acid (PLA), polycaprolactone (PCL), and polyglycolic acid (PGA). Alternatively, the biodegradable polymer may be a co-polymer comprising one or more above mentioned polymers; e.g., PCL/PLA copolymer.

According to the invention, each of the photosensitizer has one to four biodegradable polymers covalently bound thereto.

For the purpose of discussion, two illustrative examples of photosensitizer conjugates 130 are also provided in Figure 1A. The photosensitizer conjugate 130A is a porphyrin-based conjugate in which four polylactide (PLA) arms 134 are attached to a porphyrin derivative 132A at the para position of the phenyl moieties; hence, this conjugate is referred to as 4-armed porphyrin-polylactide (PPLA) conjugate in the present disclosure. On the other hand, the photosensitizer conjugate 130B is a chlorin-based conjugate which has four PLA 134 arms covalently bound to the meta-position of phenyl moieties 132B of the chlorin derivative; hence, the conjugate 130B is referred to as 4-armed chlorin-polylactide (CPLA) conjugate herein.

Structurally, the shell 120 has an aggregation number of about 2,000-15,000; preferably, 4,000-10,000. For example, the aggregation number may be about 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5500, 6000, 6500, 7000, 7500, 8000, 8500, 9000, 9500, 10000, 10500, 11000, 11500, 12000, 12500, 13000, 13500, 14000, 14500, or 15000.

Also, the particle size of the photosensitizer particles 100 is measured using dynamic light scattering (DLS), as well as small angle neutron scattering (SANS). As determined by DLS, the photosensitizer particles 100 has a diameter (also known as the hydrodynamic radius) of about 20-200 nm; preferably, about 30-100 nm. For example, the diameter of photosensitizer particles 100 is about 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 nm. The radius of the core 110, as determined by SANS, is about 10-100 nm on average; preferable; 20-50 nm. Specifically, the core radius of photosensitizer particles 100 is about 10, 15, 20, 25, 30, 35, 40, 45, 50, 55, 60, 65, 70, 75, 80, 85, 90, 95, or 100 nm. The thickness of the shell 120 is also determined by SANS, and ranges from about 10-100 nm on average; preferable; 20-50 nm. Specifically, the shell thickness of photosensitizer particles 100 is about 10, 15, 20, 25, 30, 35,40,45, 50, 55, 60, 65, 70, 75, 80, 85, 90, 95, or 100 nm.

DLS is also used to determine the polydispersity index of the photosensitizer particles 100 prepared from the assembly of a plurality of photosensitizer conjugates 130. According to various embodiments of the present disclosure, the photosensitizer particles 100 have a polydispersity index of about 0.05-0.3, and preferably, about 0.15-0,25. Specific examples of the PDI value is about 0.05, 0.06, 0.07, 0.08, 0.09, 0.1, 0.11, 0.12, 0.13, 0.14, 0.15, 0.16, 0.17, 0.18, 0-19, 0.2, 0.21, 0.22, 0.23, 0.24, 0.25, 0.26, 0.27, 0.28, 0.29, or 0.3.

According to certain embodiments of the present disclosure, the photosensitizer particle 100 is suitable for use in medical imaging procedures as a contrast agent for sonographic imaging. For example, the core 110 of the photosensitizer particle 100 may be filled with an echogenic contrast-enhancing material, such as gas or a mixture of gas and liquid. Examples of the gas composition include, but are not limited to, air, oxygen, and fluorocarbon gases. Alternatively, the gas may be a mixture containing at least two of the above-mentioned gas compositions. Specifically, the fluorocarbon gases may be any of CF₄ (tetrafluoromethane), C₂F₆(hexafluoroethane), C₃F₆ (hexafluoropropylene), G₃F₈ (octafluoropropane), C₄F₆ (1,3-hexafluorobutadiene), C₄F₈ (octafluorocyclobutan), C₄F₁₀ (perfluorobutane), and C₅F₈ (perfluorocyclopentene),

In certain embodiments, the photosensitizer particle 100 that has an echogenic contrast-enhancing material loaded therein is used in ultrasonography-based method to image a body part of a subject. Generally, the method comprises an administration step and an imaging step. In the administration step, an imaging composition comprising the photosensitizer particle contrast agent 100 according to embodiments of the present disclosure is administered to the subject. In the imaging step, the body part of interest is imaged by sonographic imaging.

Alternatively, or additionally, the photosensitizer particle 100 can be used as therapeutic agents. For example, the photosensitizer particle 100 is used in PDT to treat a subject suffered from a disease. Also, the photosensitizer particle 100 may be used to treat hypoxia. According to various embodiments of the present disclosure, the core 110 of the photosensitizer particle 100 may be filled with gas, liquid or a combination of both. In certain embodiments, the core 110 may be filled with oxygen to increase the efficacy of PDT at the oxygen-deficient tumor site. Optionally, in the case where the core 110 has liquid therein, the liquid may comprise at least one oxygen carrier such as the hemoglobin-based oxygen carrier or perfluorocarbon-based oxygen carrier to increase the oxygen-carrying capacity of the photosensitizer particle 100.

Illustrative examples of hemoglobin-based oxygen carriers include, unmodified cell-free hemoglobins, cross-linked hemoglobins, polymerized hemoglobins, recombinant hemoglobins, liposome-encapsulated hemoglobins, hemoglobin-polysaccharide conjugates, and hemoglobin analogues. Examples of perfluorocarbons suitable for use as oxygen carriers include, perfluorodecalin, perfluoromethyldecaline, perfluorocyclohexanes, perfluorotrimethylcyclohexane, perfluoroadamantane, perfluoromethyladamantane, perfluorodimethyladamantane, perfluorobicyclodecane, diphenyldimethylsiloxane, perfluoromethyl decahydroquinoline, perfluoropentane, perfluorohexane, perfluoro-n-octane, perfluorodecane, perfluorononane, octafluoropropane, perfluorodichlorooctane, perfluorohexyl bromide, perfluorooctyl bromide, perfluorodecyl bromide, bisperfluorobutylethylene, perfluorofluorene, perfluoroperhydrophenanthrene, perfluorobutylamine, and perfluorotripropylamine.

According to various embodiments of the present disclosure, a method for treating a subject suffered from a disease using photodynamic therapy comprises administering a pharmaceutical composition comprising an effective amount of the photosensitizer particle 100 to a disease site of the subject, and then irradiating the disease site with a light of appropriate wavelength and intensity for a time sufficient to elicit a therapeutic effect at the disease site.

In optional embodiments, it is possible to monitor and track the delivery of the photosensitizer particle 100, once it is administered to the subject, using an ultrasonography-based method.

In practice, the irradiation is often applied after a suitable period of several minutes to days, which allows the photosensitizer particles to accumulate in the disease site. According to various embodiment of the present disclosure, the waiting period is about 0.5 to 48 hours; preferably, about 1 to 36 hours; and more preferably, about 2 to 24 hours. Specifically, the waiting period is about 0.5, 1, 1.5, 2, 2.5, 3, 3.5, 4, 4 5, 5, 5.5, 6, 6.5, 7, 7,5, 8, 8.5, 9, 9.5, 10, 10-5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, or 48 hours after the administration of the present pharmaceutical composition.

Generally, the wavelength suitable for use in the present method may depend on several factors, such as the depth of the targeted disease site, and the structure or property of the photosensitizer or the photosensitizer particle. According to optional embodiments of the present disclosure, the wavelength suitable for activating the photosensitizer is about 550 to 750 nm; preferably, about 650 to 700 nm. For example, the wavelength may be about 550, 560, 570, 580, 590, 600, 610, 620, 630, 640, 650, 660, 670, 680, 690, 700, 710, 720, 730, 740, or 750 nm.

As could be appreciated, the light intensity, as well as the irradiation time, could be adjusted according to the tolerability of the subject, the property of the photosensitizer or the photosensitizer particle, and the desired effect of the treatment. In various embodiment of the present disclosure, the light intensity for the irradiation is about 20 to 200 J/cm². Specifically, the light intensity is about 20, 30, 40, 50, 60, 70, 80, 90, 100, 110, 120, 130, 140, 150, 160, 170, 180, 190, or 200 J/cm². According to certain embodiments of the present disclosure, the irradiation time is about 100-1000 seconds.

According to various embodiment of the present disclosure, the pharmaceutical composition comprises about 1 to 1000 mg photosensitizer particles per 1 liter of the pharmaceutical composition. For example, the concentration of the photosensitizer particles may be about N mg/L, in which N is any integral from 1 to 1000.

The present treating method is a photodynamic therapy suitable for treating various diseases. Examples of such diseases include non-small-cell lung cancer, prostate cancer, esophageal cancer, skin cancer, breast cancer, bladder cancer, pancreatic cancer, Karposi's sarcoma, retinoblastoma, age-related macular degeneration, psoriasis, arthritis and photoangioplasty of peripheral arterial disease.

The present method is particular advantageous in treating hypoxic malignant tumors because the photosensitizer particles 100 are gas-ioaded or contain oxygen carriers, and therefore, are capable of delivering oxygen to the hypoxic tumor cells. The present method is further advantageous in that it provides concurrent delivery of oxygen and photosensitizers, which improves the efficacy of the photodynamic therapy. Further, the absorption of the photosensitizer particles 100 at the treatment site could be observed and monitored with sonographic imaging so as to ensure the optimal oxygen saturation in tumor tissue.

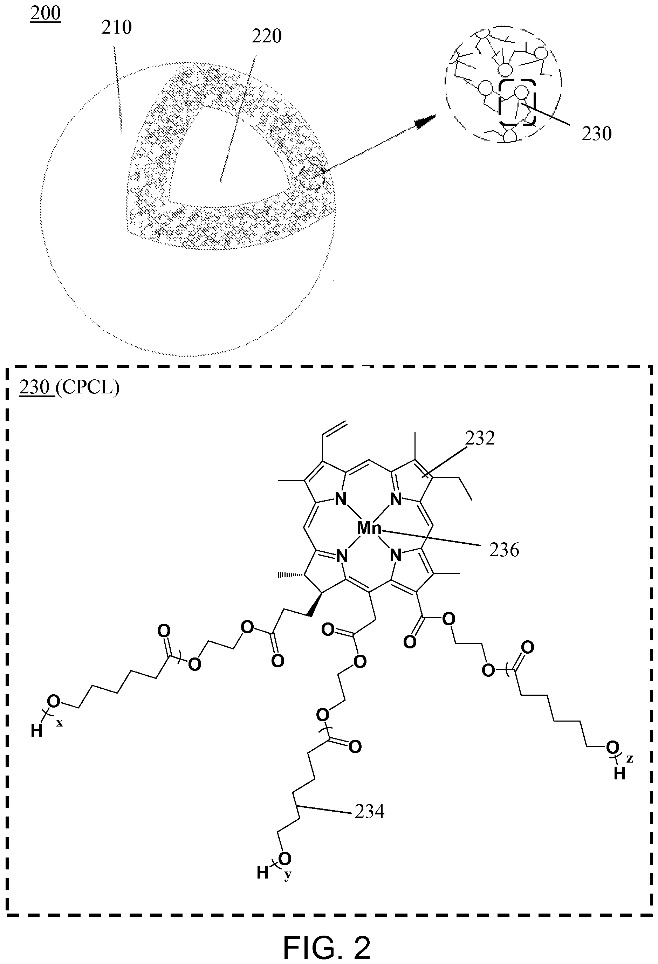

According to some other embodiments of the present disclosure, the photosensitizer particle is suitable for use in medical imaging procedures as a contrast agent. Figure 2 schematically depicts the structure of photosensitizer particles for use in MRI.

Referring to Figure 2, the photosensitizer particle 200 is suitable for use as a contrast agent in MRI procedures. Similar to the photosensitizer particle 100 of Figure 1A, the photosensitizer particle 200 is a hollow structure which comprises a core 210 and a shell 220 encasing the core 210. The shell 220 consists essentially of a plurality of photosensitizer conjugates 230. Each photosensitizer conjugate 230 consists of a photosensitizer 232 and one or more biodegradable polymer 234 covalently bound to the photosensitizer 232. Specifically, the photosensitizer conjugate 230 exemplified in Figure 2 is a 3-armed chlorin-polycaprolactone (CPCL) conjugate.

Photosensitizer particle 200 is different from photosensitizer particle 100 at least in that the photosensitizer 232 of at least one photosensitizer conjugate 230 comprises a magnetic contrast-enhancing material 236.

According to various embodiments of the present disclosure, the magnetic contrast-enhancing material 236 is a paramagnetic ion which is chelated to the photosensitizer 232. Examples of such paramagnetic ion include, but are not limited to, manganese (II), manganese (III), gadolinium (III), iron (III), iron (II), chromium (III), cobalt (II), nickel (II), copper (II), neodymium (III), samarium (III), ytterbium (III), vanadium (II), terbium (III), dysprosium (III), holmium (III) and erbium (III).

As could be appreciated, the above discussions regarding the composition and structure of the photosensitizer particle 100 are equally applicable to the photosensitizer particle 200 here. Hence, detailed descriptions regarding the photosensitizer particle 200 are omitted herein for the sake of brevity and clarity.

In certain embodiments, the photosensitizer particle 200 is used in MRI-based method to image a body part of a subject. Generally, the method comprises an administration step and an imaging step. In the administration step, an imaging composition comprising the photosensitizer particle contrast agent 200 according to embodiments of the present disclosure is administered to the subject. In the imaging step, the body part of interest is imaged by magnetic resonance imaging.

Like photosensitizer particle 100, photosensitizer particle 200 may also be used as therapeutics. For example, the core 210 may be filled with gas or a mixture of gas and liquid, so that the photosensitizer particle 200 can be used in photodynamic therapy or to threat hypoxia. Optionally, the core 210 may contain one or more oxygen carriers to increase the oxygen-carrying capacity of the photosensitizer particle 200.

In addition to the photosensitizer particles 100 or 200, the imaging and/or pharmaceutical composition of the present disclosure may further comprise a pharmaceutically-acceptable excipient (or carrier). The choice of a pharmaceutically acceptable excipient to be used in conjunction with the present photosensitizer particles is basically determined by the way the composition is to be administered.

According to one optional embodiment of the present disclosure, the composition may be administered parenterally. In this case, the present photosensitizer particles may be formulated into liquid compositions, which are sterile solutions, or suspensions that can be administered by, for example, intravenous, intramuscular, subcutaneous, or intraperitoneal injection. The solution or suspension is preferably isotonic with the body fluid of the recipient. Such formulations may be prepared by suspending the photosensitizer particles in water containing physiologically compatible substances such as sodium chloride, glycine, and the like, and having a buffered pH compatible with physiological conditions to produce an aqueous solution, and rendering said solution sterile. Other diluents or solvent suitable for manufacturing sterile injectable solution or suspension include, but are not limited to, 1,3-butanediol, mannitol, water, and Ringer's solution. Fatty acids, such as oleic acid and its glyceride derivatives are also useful for preparing injectables, as are natural pharmaceutically-acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation.

For oral administration, the photosensitizer particles of the present application may be formulated into aqueous suspensions and/or elixirs in which the photosensitizer particles are combined with various sweetening or flavoring agents, coloring matter or dyes, and if so desired, emulsifying and/or suspending agents as well, together with diluents such as water, ethanol, propylene glycol, glycerin and a combination thereof.

When topical administration is desired, a wide variety of dermatologically acceptable inert excipients well known to the art may be employed. The topical compositions may include liquids, creams, lotions, ointments, gels, sprays, aerosols, skin patches, and the like. Typical inert excipients may be, for example, water, ethyl alcohol, polyvinyl pyrrolidone, propylene glycol, mineral oil, stearyl alcohol and gel-producing substances. All of the above dosages forms and excipients are well known to the pharmaceutical art. The choice of the dosage form is not critical to the efficacy of the composition described herein.

The following Examples are provided to elucidate certain aspects of the present invention and to aid those of skilled in the art in practicing this invention. Without further elaboration, it is believed that one skilled in the art can, based on the description herein, utilize the present invention to its fullest extent.

### Example

### Materials

3-(4,5-dimethylthiazol-2-yl)-2,5-diphenyl tetrazolium bromide (MTT), dimethylsulfoxide (DMSO), and tetrahydrofuran (THF) were obtained from Sigma (Sigma-Aldrich, Germany and USA). Sodium phosphate dibasic (Na₂HPO₄) and sodium chloride (NaCl) were obtained from Tedia (Tedia, Fairfield, OH, USA); potassium chloride (KCI) and potassium dihydrogenphosphate (KH₂PO₄) from Showa (Showa, Japan); sodium bicarbonate (NaHCO₃) from Scharlau (Scharlau, USA); and triethylamine (TEA) from Baker (J.T. Baker, Phillipsburg, NJ, USA). For cell culture studies, Dulbecco's Modified Eagle Medium (DMEM), MEM, fetal bovine serum (FBS), and 0.25% trypsin-EDTA were purchased from Gibco (Gibco-BRL, Gaithersburg, MD, USA). Penicillin-streptomycin-neomycin solution and 10% formalin were purchased from Sigma-Aldrich (Sigma-Aldrich, ST. Louis, MO, USA). N,N-Dimethylethane-1,2-diamine and 2-hydroxybenzaldehyde were purchased from Showa and used without further purification.

### Example 1

### Preparation and Characterization of PPLA, CPLA and CPCL Conjugates

### Example 1.1: PPLA

The whole preparation process was conducted under dry nitrogen. Solvents and reagents were dried by refluxing for at least 24 hours over sodium/benzophenone (for hexane, toluene, and THF) or anhydrous magnesium sulphate (for benzyl alcohol). L-Lactide was recrystallized from a toluene solution prior to use.

The catalyst for the ring-opening polymerization (ROP) of 4-armed porphyrin-PLA (PPLA) conjugate was prepared as follows. A mixture of N,N-dimethylethane-1,2-diamine (8.8 g, 100 mmol), 2-hydroxybenzaldehyde (12.2 g, 100 mmol), and HCI (aq.) (35%, 0.30 mL) was stirred in absolute THF (30 mL) for 1 day. Volatile materials were removed under vacuum to give 2-[(2-dimethylamino-ethylimino)methyl]phenol (DAIP-H; yellow oil; yield: 18.2 g; 95%). The mixture of DAIP-H (1.92 g, 10 mmol) with Ca(OMe)₂ (0.51 g, 5 mmol) was stirred in toluene/THF (20/5 ml) at 100°C for 3 days in a sealed tube, and subsequently surplus Ca(OMe)₂ was removed by filtration. Volatile materials were removed under vacuum to yield yellow powder. The powder was washed with hexane (30 mL) twice to remove excess ligand and the yellow powder (calcium di-2-[(2-dimethylamino-ethylimino)methyl]phenol ([(DAIP)₂Ca]₂) was obtained after filtration (yield: 1.27 g; 60%).

The ring-opening polymerization for the synthesis of the 4-armed PPLA conjugate was as follows. The porphyrin derivative, meta-tetra-(*p-*hydroxymethylphenyl) porphyrin (m-THMPP) (BnOH) (0.001 g, 1.36 ×10⁻³ mmol), and [(DAIP)₂Ca]₂ catalyst (0.004 g, 4.74 ×10⁻³ mmol) were dissolved in THF (10 mL). The reaction mixture was stirred for 1 hour, and then L-lactide (0.078 g, 0.54 mmol) was added for ROP at room temperature (about 23-28°C) for 6 hours. Volatile materials were removed *in vacuo,* and the residue was re-dissolved in THF (5 mL). The mixture was then quenched by the addition of an aqueous acetic acid solution (0.35 N, 10 mL), and the polymer was precipitated on pouring into n-hexane (40 mL) as white crystalline solids (PPLA; yield: 0.05 g; 64%).

The structure and the percentage of the actual molar composition of the product were determined by ¹H NMR (Varian Unity Inova-600, Palo Alto, CA, USA) in CDCl₃ (deuterated chloroform) with tetramethyl silane as the standard. The average M_{w} and polydispersity of PPLA were calculated by gel permeation chromatography (GPC) with an RID-6A refractive index detector (Shimadzu, Kyoto, Japan) and an ultrastyragel column. The mobile phase was THF eluent at a flow rate of 1 mL/min. Calibration was accomplished with monodispersed polystyrene standards.

The resultant PPLA conjugates were termed as PPLA1. The molecular weight of each PLA chain on PPLA1, obtained from the ¹H NMR analysis, was 13,140 and the polydispersity, obtained from GPC, was 1.66 (M_{w}/Mₙ: 23,600/14,200). Thus, the hydroxyl-terminated porphyrin can successfully act as an initiator of ROP for lactide.

Various PPLA conjugates with different molecular weights (e.g., 6,000-130,000 Da) were prepared by adjusting the amount of L-lactide (e.g., 8.35-2000 µmol). Another PPLA conjugate (PPLA2) prepared according to this example had a molecular weight of about 53,300 Da.

### Example 1.2: CPLA

In the present example, the 4-armed chlorin-PLA (CPLA) conjugate was synthesized from *meta*-tetra-3-hydroxymethyl phenyl chlorin (m-THMPC).

The m-THMPC was synthesized as follows. First, NaBH₄ (0.425 g, 9.25 mmol) was added at -5°C with continuous stirring for 30 minutes to a solution of dialdehyde 1 (5 g, 37 mmol) in a mixture of dry EtOH (75 ml) and THF (100 ml). The mixture was then stirred for 10 hours, and the temperature was maintained at about 0 to -5°C while stirring. The reaction mixture was then neutralized with 2M HCI to pH 5 before the solvents were evaporated. Thereafter, water (200 mL) was added to the residue which was then extracted with AcOEt. The combined organic extracts were dried with MgSO₄, and the solvent was evaporated. The product was purified by column chromatography using an AcOEt-hexane (30/70) mixture of solvents. Hydroxymethyl aldehyde was obtained as a colorless liquid and the yield was 2.7 g (54%).

Then, to a solution of 3-Hydroxy methyl benzaldehyde (0.487 g, 3.576 mmol), dicyclohexylcarbodiimide (DCC; 0.885 g, 4.291 mmol), and 4-N,N-dimethylaminopyridine (DMAP; 0.0436 g, 0.37 mmol) in dry CH₂Cl₂ was added acetic acid (2.0 mL). The mixture was stirred at room temperature under N₂ for 12 hours to allow the formation of a precipitate. The precipitate was removed by filtration and the filtrate was concentrated and purified with silica gel column chromatography (ethylacetate-hexane 15/85) to give 0.450 g (70%) of ester (3-acetoxymethylbenzaldehyde) as a colorless liquid.

A solution of 3-acetoxymethylbenzaldehyde (1.255 g, 7.044 mmol) and 3-acetoxymethyl phenyl dipyrromethane (2.073 g, 7.044 mmol) in CH₂Cl₂ (750 ml) was purged with nitrogen for 30 minutes, and then trifluoroacetic acid (TFA) (0.341 ml, 4.443 mmol) was added. The mixture was stirred for 3 hours at room temperature, and then 2,3-dichloro-5,6-dicyano-1,4-benzoquinone (DDQ ; 3.199, 14.088) was added. After the mixture was stirred at room temperature for an additional 15 minutes, the reaction was quenched by adding triethylamine (1 mL). The solvent was removed, and the residue was purified with silica gel column chromatography using methanol-dichloromethane (99:1) as the eluent to give 0.954 g (yield, 30%) of tetra-3-acetoxymethyl phenyl porphyrin.

Next, tetra-3-acetoxymethyl phenyl porphyrin 1.75g 1.93 mmol) and anhydrous potassium carbonate (2.4g 17.44 mmol) were dissolved in dry pyridine (50 ml) before the addition of p-toluenesulfonhydrazide (0.72g 3.87 mmol) in dry pyridine. The mixture was then heated at 100-105°C under nitrogen for 24 hours. During the heating period, further quantities of p-toluenesulfonhydrazide (0.72g 3.87 mmol in dry pyridine) were added after 2, 4, 6 and 8 hours, respectively. After cooling to room temperature, the mixture was treated with ethyl acetate/water (2v/1v) and then heated at 100°C for 3 hours. After cooling to room temperature, the organic phase was separated and washed twice with aqueous HCI (2 M), twice with water and then saturated with aqueous sodium hydrogen carbonate solution. The presence of chlorin and bacteriochlorin in the solution was controlled by UV-visible spectroscopy (bands at 651 and 738 nm, respectively). o-Chloranil (0.72g 3.87 mmol) was slowly added to the stirred organic solution at room temperature until the absorption peak of bacteriochlorin species disappeared. The solution was then washed with aqueous solution of NaHSO₃ (5 %), water and dried over sodium sulfate. The filtered solution was concentrated under vacuum, and the crude product was purified by silica gel column chromatography with 30% EA. The tetra-3-acetoxymethyl phenyl chlorin (1.275 g; yield, 73%) was obtained as mauve powder.

Thereafter, a solution of lithium hydroxide monohydrate (1.85 g 44.1 mmol) in water (20 ml) was added to a solution of the above-mentioned chlorin acetoxy ester (1 g, 1.1 mmol) in THF (40 ml) and the mixture was stirred at room temperature for 24 hours. The pH was adjusted to about 1 with hydrochloric acid (2 M). Water and ethyl acetate were then added, and the aqueous phase was extracted with ethyl acetate. The combined organic phases were washed with water and brine, dried over magnesium sulfate and concentrated to give meta-tetra-3-hydroxymethyl phenyl chlorin (mTHMPC; 0.640 g; yield, 80%).

For the synthesis of 4-armed chlorin-PLA (CPLA) conjugate, mTHMPC (0.001 g, 1.67 ×10-3 mmol) and stannous octoate (SnOct₂) catalyst (0.324 g, 0.8 mmol) were dissolved in toluene (10 mL). The reaction mixture was stirred at 100°C for 1 hour, and then L-lactide (0.012 g, 8.35 ×10⁻² mmol) was added for ROP at 100°C for 5 hours before the reaction mixture was cooled down to room temperature. The mixture was then quenched by the addition of ethanol (10 mL), and the polymer was precipitated on pouring into n-hexane (50 mL) as reddish-brown solids (yield: 0.01 g; 75%). The resultant CPLA conjugates were termed herein as CPLA2, which had a molecular weight of about 7,200 Da. Various CPLA conjugates with different molecular weights (e.g., 6,000-130,000 Da) were prepared by adjusting the amount of L-lactide (e.g., 8.35-2000 µmol). For example, other CPLA conjugates prepared in this example included CPLA1 (Mn: 6,000 Da); CPLA3 (Mn: 15,000 Da); and CPLA4 (Mn: 130,000 Da).

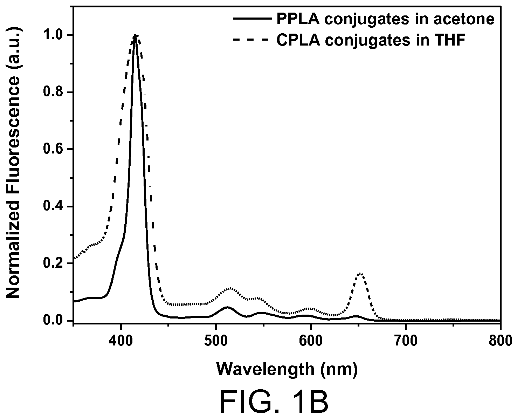

The fluorescence spectra of PPLA1 (Example 1.1) and CPLA2 (Example 1.2) were measured with Spectrofluorometer (JASCO FP-6300, Tokyo, Japan) at room temperature. The results are illustrated in Figure 1B. As could be seen in Figure 1, the absorbing range of the porphyrin-based PPLA conjugate was quite limited with a maximum absorption of about 405-430 nm. The short absorption wavelength of porphyrin has limited its application in PDT application, because the light with shorter wavelength may not penetrate far enough to reach the target site. In contrast, the chlorin-based CPLA conjugate had two absorption peaks at 405-430 nm and 650-680nm, respectively. The additional absorption peak of the CPLA conjugate allows for its application in PDT.

The PPLA conjugate is also different from the CPLA conjugate in the position of the PLA side chains. Specifically, the PLA side chains of PPLA are attached to the para-position of the phenyl group (see, 130A of Figure 1A); whereas the PLA side chains are attached to the meta-position of the phenyl group (see, 130B of Figure 1A).

### Example 1.3: CPCL

The 3-armed chlorin-PCL (CPCL) conjugate was synthesized by ring-opening polymerization as follows. The chlorin (0.3 g, 0.5 mmol) and sodium carbonate (1.05 g, 10 mmol) were dissolved in THF (40 mL). The reaction mixture was stirred for 10 minutes at 0°C, and then thionyl chloride (1 mL, 13.90 mmol) was added, and the reaction mixture was stirred and warmed up to room temperature for 3 hours. Excessive amount of ethylene glycol (2 mL, 30 mmol) was added for reaction for 1 day. Volatile materials were removed in vacuo, and the residue was re-dissolved in water (30 mL), and then extracted three times with carbon dichloride (20 mL). The organic layer was collected and dried in vacuo to give green powders. The green powders, stannous octoate (0.324 g, 0.8 mmol), and 2,2'-ethylidene-bis(4,6-di-tert-butylphenol) (EDBP-H2) (0.351 g, 0.8 mmol) were dissolved in toluene (10 mL). The reaction mixture was stirred at 100°C for 1 hour, and then ε-caprolactones (4.56 mL, 41.04 mmol) was added for ROP at 100°C for 5 hours before the reaction mixture was cooled down to room temperature. The mixture was then quenched by the addition of ethanol (10 mL), and the polymer was precipitated on pouring into n-hexane (50 mL) as dark green solids (CPCL; yield: 4.01 g).

The resultant CPCL conjugates were termed as CPCL1 herein. The structure and the percentage of the actual molar composition of the CPCL1 were determined by ¹H NMR (Varian Unity Inova-600, Palo Alto, CA, USA) in acetone with tetramethyl silane as the standard. The average Mw and polydispersity of CPCL1 were calculated by gel permeation chromatography (GPC) with an RID-6A refractive index detector (Shimadzu, Kyoto, Japan) and an ultrastyragel column. The mobile phase was THF eluent at a flow rate of 1 mL/min. Calibration was accomplished with monodispersed polystyrene standards. The polydispersity, obtained from GPC, was 1.14 (Mw/Mn: 6,031/5,282).

Various CPCL conjugates with different molecular weights (e.g., 9,000-70,000 Da) were prepared by adjusting the amount of ε-caprolactones (e.g., 80-680 mmol). Other CPCL conjugates prepared in this example included, CPCL2 (Mw/Mn: 9,772/12,792), CPCL3 (Mw/Mn: 14,340/22,269), CPCL4 (Mw/Mn: 6,917/9,013), CPCL5 (Mw/Mn: 41,126/69,808).

To prepare manganese (III) chelated CPCL (Mn-CPCL) conjugate, one equivalent of CPCL1 conjugate was dissolved in 60 mL of THF and mixed with 20 mL of 120 equivalent manganese acetate methanol solution. The reaction was carried out under 90°C of reflux for a total of 24 hours. In addition, 20 mL of methanol was added into the reaction solution twice respectively at 1 and 13.5 hours after the reaction was executed. After the reaction solution was cooled down to room temperature, this solution was condensed by rotavapor and then dissolved in dichloromethane. The sample was purified by extracting with deionized water for three times, and afterwards the collected organic layer products were dialyzed against 3 liters of double distilled (d.d.) water. Purified product was dried using freeze drying.

### Example 2

### Preparation and Characterization of Native and Gas-loaded Photosensitizer Particles

Two different approaches (i.e., solvent evaporation and double emulsion) were adopted in the present example for the preparation of photosensitizer particles.

### Example 2.1: Solvent Evaporation

To obtain native CPCL, CPLA or PPLA photosensitizer particles that are not loaded with gas, PPLA, CPLA or CPCL conjugates from Example I above were dissolved in acetone or THF. The conjugate solution was then drop-wisely added into double distilled (d.d.) water at room temperature with stirring to allow for the self-assembly of native photosensitizer particles. For CPCL conjugates, the conjugate solution was pipetted with the tip of the pipetman above (examples CN1, CN2) or under (example CN3) the d.d. water. As to CPLA and PPLA conjugates, the conjugate solution was added using a syringe pump above the d.d. water. Summarized in Table 1 are the manufacturing parameters for the native photosensitizer particles according to various embodiments of the present disclosure.

The average hydrodynamic radius (R_{H}; in Z-average value) and polydispersity (PDI) of the native photosensitizer particles were measured using dynamic light scattering (DLS, Nano ZS90). Briefly, the aqueous solution containing photosensitizer particles was evaluated at 25 °C at a fixed angle of 90° for the DLS measurement, and the results are also summarized in Table 1.

It should be noted that under same manufacturing condition, the average diameter of the CPLA photosensitizer particle was larger than that of the PPLA photosensitizer particle. For example, except for the solvent, the photosensitizer particles of examples LN1 to LN4 and the photosensitizer particles of examples of PN4 were prepared using the same manufacturing factors (e.g., amount, stir speed, and drop rate); yet, the average diameters of LN1 to LN4 photosensitizer particles were much greater than the PN4 photosensitizer particles.

**Table 1**

| **No** | Polymer (mg) | Solvent (mL) | H₂O (mL) | Stir (rpm) | Drop rate (mL/hr) | R_{H} (d nm) | PDI |
|---|---|---|---|---|---|---|---|
| **CN1** | CPCL5, 2 | Acetone, 3 | 5 | 450 | 0.25 | 62.86±0.335 | 0.108±0.009 |
| **CN2** | CPCL5, 10 | THF, 5 | 50 | 450 | 0.25 | 114.1±0.8718 | 0.184±0.017 |
| **CN3** | CPCL5, 10 | THF, 5 | 50 | 200 | 0.5 | 120.8±1.305 | 0.147±0.023 |
| **LN1** | CPLA1, 10 | THF, 5 | 10 | 450 | 0.25 | 140.1±0.5508 | 0.080±0.012 |
| **LN2** | CPLA2, 10 | THF, 5 | 10 | 450 | 0.25 | 163.7±0.6245 | 0.113±0.020 |
| **LN3** | CPLA3, 10 | THF, 5 | 10 | 450 | 0.25 | 157.5±0.8021 | 0.122±0.016 |
| **LN4** | CPLA4, 10 | THF, 5 | 10 | 450 | 0.25 | 174.7±0.4933 | 0.080±0.014 |
| **LN5** | CPLA2, 10 | THF, 5 | 10 | 450 | 0.25 | 194.5±2.146 | 0.170±0.013 |
| **PN1** | PPLA1, 2 | Acetone, 1 | 5 | 450 | 0.25 | 103.3±0.9192 | 0.186±0.026 |
| **PN2** | PPLA1, 10 | Acetone, 1 | 5 | 450 | 0.25 | 72.19±0.5422 | 0.124±0.009 |
| **PN3** | PPLA1, 2 | Acetone, 1 | 10 | 450 | 0.25 | 67.23±0.1644 | 0.172±0.011 |
| **PN4** | PPLA1, 10 | Acetone, 5 | 50 | 450 | 0.25 | 79.63±0.9052 | 0.168±0.006 |
| **PN5** | PPLA1, 10 | Acetone, 5 | 50 | 450 | 0.25 | 89.78±0.2751 | 0.142±0.015 |
| **PN6** | PPLA1, 10 | Acetone, 10 | 50 | 450 | 0.5 | 73.17±1.773 | 0.142±0.005 |
| **PN7** | PPLA1, 25 | Acetone, 10 | 12.5 | 450 | 0.25 | 89.68±0.4223 | 0.126±0.001 |

For the preparation of gas-loaded photosensitizer particles, gas was pumped into d.d. water while the conjugate solution was drop-wisely added into d.d. water under stir. After the conjugate solution was depleted, the volatile solvent (i.e., acetone or THF) was removed by pumping gas into the mixture for at least 30 minutes. Summarized in Table 2 are the manufacturing parameters for the gas-loaded photosensitizer particles prepared by the solvent evaporation approach. Physical properties of the gas-loaded photosensitizer particles were measured as described above, and the results are also given in Table 2.

**Table 2**

| **No** | Polymer (mg) | Solvent (mL) | H₂O (mL) | Stir (rpm) | Drop rate (mL/hr) | R_{H} (d nm) | PDI |
|---|---|---|---|---|---|---|---|
| **CA1** | CPCL5, 5 | Acetone, 5 | 25 | 100 | 0.5 | 59.11±0.2899 | 0.262±0.014 |
| **CA2** | CPCL5, 6 | Acetone, 6 | 30 | 150 | 0.5 | 67.15±0.2759 | 0.171±0.006 |
| **CA3** | CPCL5, 10 | THF, 5 | 50 | 450 | 0.25 | 83.31±0.2787 | 0.157±0.007 |
| **CA4** | CPCL5, 10 | THF, 5 | 50 | 200 | 0.5 | 85.60±0.5622 | 0.128±0.013 |
| **LA1** | CPLA1, 10 | THF, 5 | 10 | 450 | 0.25 | 97.31±0.6329 | 0.166±0.013 |
| **LA2** | CPLA2, 10 | THF, 5 | 10 | 450 | 0.25 | 135.0±2.757 | 0.132±0.013 |
| **LA3** | CPLA3, 10 | THF, 5 | 10 | 450 | 0.25 | 140.3±0.4359 | 0.069±0.014 |
| **LA4** | CPLA4, 10 | THF, 5 | 10 | 450 | 0.25 | 148.7±0.9000 | 0.105±0.004 |
| **LA5** | CPLA2, 10 | THF, 5 | 10 | 800 | 0.25 | 134.2±0.3215 | 0.104±0.001 |
| **PA1** | PPLA1, 3 | Acetone, 3 | 30 | 600 | 0.5 | 80.22±0.6669 | 0.135±0.010 |
| **PA2** | PPLA1, 2 | Acetone, 1 | 10 | 450 | 0.25 | 76.25±1.050 | 0.253±0.011 |
| **PA3** | PPLA1, 2 | Acetone, 1 | 5 | 450 | 0.25 | 60.44±0.0141 | 0.208±0.005 |
| **PA4** | PPLA1, 10 | Acetone, 10 | 100 | 450 | 0.5 | 60.29±0.260 | 0.209±0.004 |
| **PA5** | PPLA1, 10 | Acetone, 10 | 50 | 450 | 0.5 | 62.50±0.465 | 0.209±0.010 |

As could be inferred from the data in Table 2, different particle sizes could be achieved by adjusting the molecular weights of the conjugates. Take the LA1, LA2, LA3 and LA4 photosensitizer particles as an example, the particle sizes increased as the molecular weights of the conjugates increased.

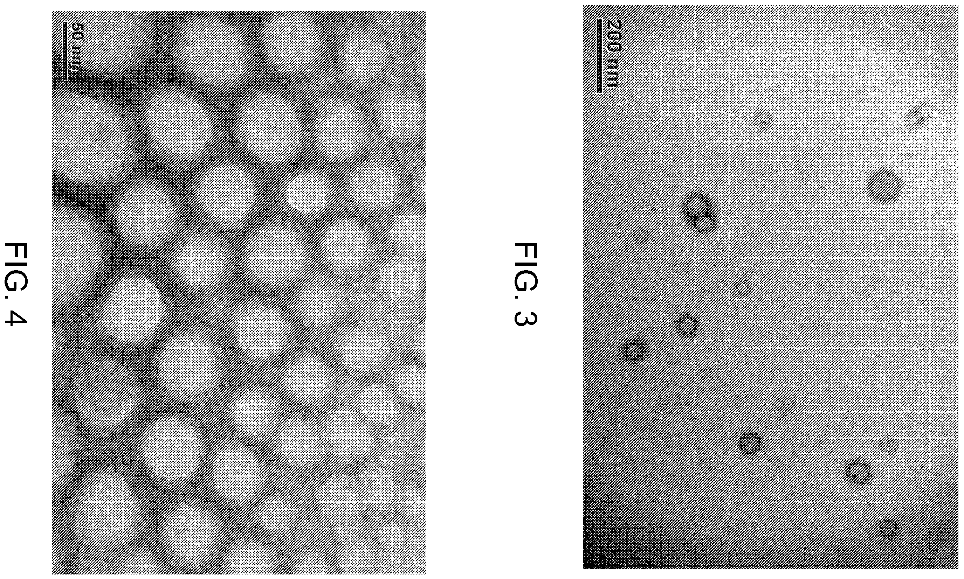

The morphology of the gas-loaded photosensitizer particles was examined using transmission electron microscopy (TEM; JEOL JEM-1400 cryo-TEM, JEOL, Ltd., Akishima, Japan). Figure 3 is a representative TEM image of gas-loaded photosensitizer particles of Example PA2 (hereinafter, gas-loaded PA2 photosensitizer particles).

### Example 2.2: Double emulsion

Here, the native CPLA photosensitizer particles were prepared by water-in-oil-in-water (W/O/W) double emulsion. Briefly, 25 mg of CPLA-1 conjugate (Example 1.2, above) was dissolved in 1 mL of dichloromethane. Then, 100 or 50 µL of 0.1 % (w/v) polyvinyl alcohol (PVA) aqueous solution was poured into the polymer solution with sonication using ultrasonic probe for 1.5 minutes to give a water-in-oil (W/O) emulsion. Afterwards, the W/O emulsion was poured into 2 mL of 5 % (w/v) PVA aqueous solution with ultrasonic probe sonication for 3 minutes to give a W/O/W emulsion. All the sonication processes were carried out on ice. Finally, the W/O/W solution was poured into 40 mL of 0.1 % (w/v) PVA aqueous solution which was then stirred with magnetic stir bar (340 rpm; room temperature) overnight to evaporate the volatile solvent (i.e., dichloromethane). Thereafter, the excess PVA in the final sample was removed by centrifugation. For centrifugation, the sample was centrifuge at 10,000 g and 4°C for 1 hour, and then the sediments were washed with fresh deionized water. The centrifugation and washing procedures were repeated at least three times to substantially remove the PVA. The resulting CPLA particles were stored at 4°C and protected from light.

Summarized in Table 3 are the manufacturing parameters for the native CPLA photosensitizer particles prepared by the double emulsion approach.

**Table 3**

| **No** | Polymer | Inner Phase (0.1% PVA, µL) | R_{H} (d nm) | PDI |
|---|---|---|---|---|
| **LW1** | CPLA2 | 50 | 235.9±5.994 | 0.480±0.101 |
| **LW2** | CPLA2 | 100 | 244.5±7.621 | 0.486±0.081 |
| **LW3** | CPLA3 | 50 | 265.0±5.704 | 0.455±0.012 |
| **LW4** | CPLA3 | 100 | 144.9±2.811 | 0.121±0.008 |
| **LW5** | CPLA4 | 50 | 261.0±3.172 | 0.409±0.008 |
| **LW6** | CPLA4 | 100 | 213.2±2.635 | 0.390±0.012 |

### 2.3: Structural analysis

The detailed structure of the photosensitizer particles was examined using small angle neutron scattering (SANS; CG2-SANS; Oak Ridge, Tennessee, USA). Based on the TEM finding in Figure 3, the SANS data were analyzed with two models describing the possible structures: polydisperse core-shell micelles (hereinafter, the micelle model) and polydisperse core-shell spherical vesicles (hereinafter, the vesicle model). The micelle model assumes the structure of a hydrophobic core composed of the assembled porphyrin surrounded by a shell of polylactide, while the vesicle model assumes that the PPLA conjugates constitute a spherical shell surrounded by and filled with D₂O.

For SANS analysis, the native PPLA photosensitizer particles that were not loaded with gas were prepared by drop-wisely adding the PPLA1 conjugate solution (2 mg in 1 mL of acetone) into D₂O at room temperature with stirring to form a solution containing 0.2 wt% native PPLA photosensitizer particles according to the method set forth in Example 2.1, above. The solution was then diluted with D₂O to produce the final solutions respectively containing 0.05 and 0.1 wt% native PPLA photosensitizer particles . The SANS experiments were performed on the CG2-SANS instrument located at the High Flux Isotope Reactor (HFIR) at Oak Ridge National Laboratory (Oak Ridge, Tennessee, USA). Neutrons with a wavelength of 4.75 Å and two sample-to-detector distances (s.d.d.) of 4 m and 18.5 m were used to cover a q range from 0.004 Å⁻¹ to 0.26 Å⁻¹. All of the 2-D raw data were properly corrected for the sample and empty cell transmissions and for the background scattering, and then the data were reduced to 1-D intensity plots as a function of q (defined as 4π/*λ* sin*θ*/2, where *λ* and *θ* are the wavelength and scattering angle, respectively).

Each SANS spectra was best fit using the program developed at NIST Center for Neutron Research, which was written in the IGOR PRO 6.0 software package. For the micelle model, the diameter determined from the best fit (20 nm) was not consistent with the results obtained from the TEM and DLS measurements. For this reason, the micelle model was negated. As to the vesicle model, the core radius and shell thickness (35 and 19 nm, respectively) from the best-fit are consistent with the DLS (hydrodynamic radius -52 nm). The radius of gyration (R_{g}) determined from the SANS data is 46.1 nm. The TEM result, as illustrated in Figure 4, validates this vesicle model. In addition, the polydispersity was approximately 0.5.

To further confirm the morphology of these native PPLA photosensitizer particles, static light scattering (SLS; BI-200SM, Brookhaven Instruments, Co., New York, USA) was employed to determine the average aggregation number of the native PPLA photosensitizer particles. Briefly, intensity traces were obtained at different angles to derive the radius of gyration (R_{g}) and the molecular mass of the native PPLA photosensitizer particles. For the SLS measurement, the incident laser beam has a wavelength at 632.7 nm and the scattered intensities of the dilute native PPLA photosensitizer particle solutions (0.015-0.05 mg/mL) were collected as a function of the scattering angles. A Zimm plot was constructed to provide the apparent weight average molecular mass (M_{w,app}) of the native PPLA photosensitizer particles. The SLS measurements were performed at 25°C in d.d. water at angles between 40° and 140°. The M_{w,app} of the native PPLA photosensitizer particles determined from the Zimm plot is about 2.615 X 10⁸ g/mol, and The aggregation number of native PPLA photosensitizer particles is about 4900. The R_{g} determined from SLS is about 48.4 nm, which is similar to that determined from SANS (about 46.1 nm); also, the shape factor (R_{g}/R_{H}) of the native PPLA photosensitizer particles is close to the typical value 1 of a hollow sphere.

Taking SANS and SLS results together, it is speculated that the structure of native PPLA photosensitizer particles is a hollow structure as a porphysome. In addition, it is supposed that the porphyrin molecules were not attracted to each other; rather, they were separated by PLA molecules in the native PPLA photosensitizer particles. This unique structure may advantageously confer enhanced photostability on native PPLA photosensitizer particles since the PLA chains may impede the photodegradation of porphyrin molecules.

### Example 3

### Ultrasonic Imaging of Gas-loaded Photosensitizer Particles

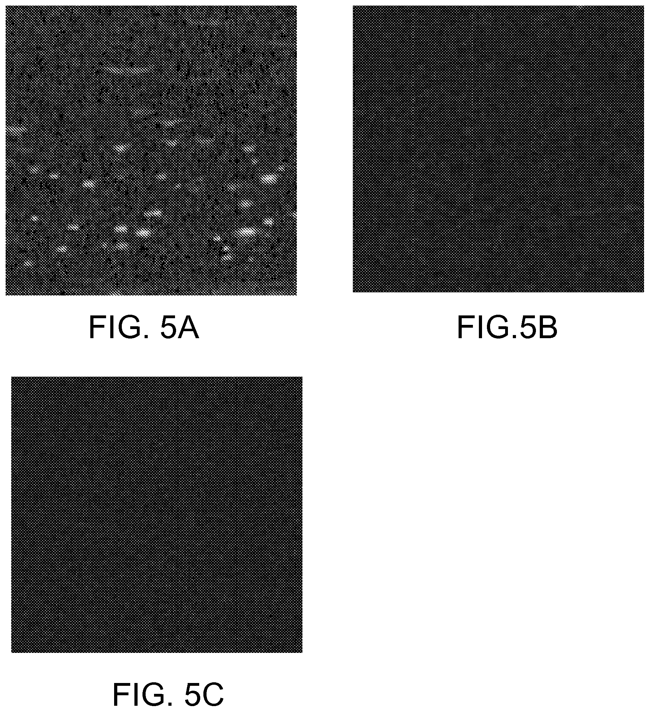

Ultrasonic imaging was conducted using a Visualsonics Vevo 770® system (VisualSonics Inc., Toronto, Ontario, Canada) operating at a scanning frequency of 40 Hz. Briefly, three agar sheets (1 wt%) were stacked vertically and immersed in deionised water. The agar sheet in the middle had pre-made holes (diameter: about 8 mm, height: about 10 mm). The three agar sheets were fixed in place with toothpicks so as to ensure that the holes were fully filled with deionised water. Thereafter, 100 µL of gas-loaded PA1 photosensitizer particles (from Example 2.1, above; the experimental group) or native PN6 photosensitizer particles (from Example 2.1, above; the control group) were mixed with water and agar gel (final concentration: about 0.75 µM) and then injected into the holes in the middle layer of the agar plate using a syringe. To perform the B-mode (2-D) ultrasonic imaging, a 40 MHz transducer was placed in the proximity of the top agar sheet and then moved horizontally to obtain scanning area of about 1 cm². Representative ultrasonic images of the experimental group, the control group and the negative control group (deionized water only) were provided in Figure 5A, Figure 5B and Figure 5C, respectively. The bright dots in Figure 5A represent the ultrasound echoes reflected by the echogenic, gas-loaded PA1 photosensitizer particles, and the brightness of each dot is dependent on the amplitude of the returned echo signal. By contrast, no bright dots are observed in either Figure 5B or Figure 5C, indicating that the native PN6 photosensitizer particles, like water molecules, are not echogenic. In view of the foregoing, the gas-loaded photosensitizer particles according to various embodiments of the present disclosure are suitable for use as a contrast agent for ultrasonic imaging.

Currently, ultrasonic contrast agents are mostly lipid-based microbubbles or nanobubbles. One major limitation of these ultrasonic contrast agents is their short blood circulation time. However, it is suggested that polymer-based bubbles can exhibit a higher stability in blood. Therefore, the present gas-loaded photosensitizer particles may provide a robust and highly stable platform for air/gas encapsulation, thereby enhancing the blood circulation time. Further, prior researches indicate that nano-sized PPLA porphysomes can passively target the tumor site, whereas microbubbles cannot. Therefore, the present gas-loaded photosensitizer particles is particular suitable for use in the imaging of tumor tissues.

### Example 4

### Cellular Uptake and Phototoxicity of Gas-loaded Photosensitizer Particles

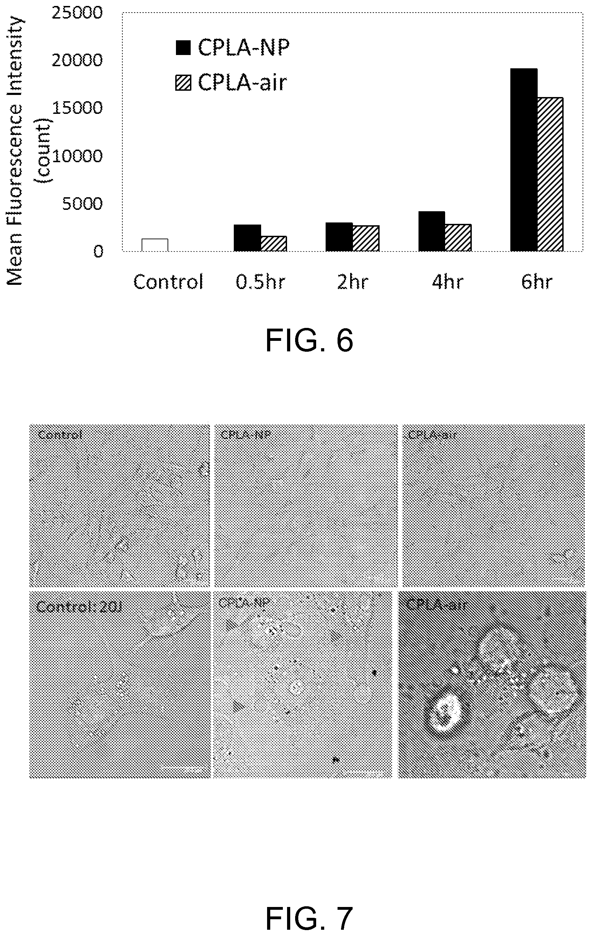

Cellular uptake of gas-loaded photosensitizer particles was investigated *in vitro.* Human non-small lung carcinoma cell line (H1299) (5 X 105 cells/dish) were incubated with 3 mL of culture medium (10% FBS, supplemented with 1% antibiotics and 1% sodium pyruvate) at 37°C for 24 hours. Stock solutions of gas-loaded LA4 photosensitizer particles (CPLA-air) and native LN4 photosensitizer particles (CPLA-NP) were respectively added into the culture medium to a final concentration of 0.5 µM. Further, the culture medium containing 15 mM of H₂O₂ was used as the positive control. Cells were then incubated for 24 hours, and the florescence intensity was analyzed by flow cytometry (Accuri™ C6, BD) before and at 0.5, 2, 4 and 6 hours after the addition of the photosensitizer particles. All the experiments were carried out in dark after photosensitizer particles were added. Results summarized in Figure 6 reveal that the cancerous H1299 cells are capable of absorbing both CPLA-air and CPLA-NP.

After cultivating with photosensitizer particles for 24 hours, cells were irradiated with light (20 J/cm²) at 661 nm. Cells before and after the light irradiation were subject to morphological analysis using a confocal microscope (Lecia, SP5). The microscopic photographs in the top panel of Figure 7 indicate that CPLA-air photosensitizer particles, as well as CPLA-NP photosensitizer particles, are absorbed by H1299 cells, as is evidenced by the red fluorescence in the cytosol. Significant change in cellular morphology, before and after light irradiation, is observed by comparing photographs in the top and bottom panels of Figure 7. Specifically, in the bottom panel of Figure 7, a plurality of membrane blebbing (indicated by arrows) are present in cells underwent photodynamic therapy. This morphology change may be associated with the death/destruction of cancerous cells.

H₂DCFDA kit (purchased from Molecular Probe) was used to evaluate the production of reactive oxygen species during photodynamic therapy using the present gas-loaded photosensitizer particles. After co-incubation with 0.5 µM of native LN4 or gas-loaded LA4 photosensitizer particles for 24 hour, cells were washed three times using 1×PBS, followed by incubation with 5 µM of H₂DCFDA for 0.5 hours. Thereafter, cells were washed three times using 1×PBS and then exposed to light irradiation (661 nm; 20 J/cm²). The fluorescence of photosensitizer particles and H₂DCFDA were observed using Confocal microscope (Lecia, SP5) with 405 and 488 nm of laser excitation, respectively.

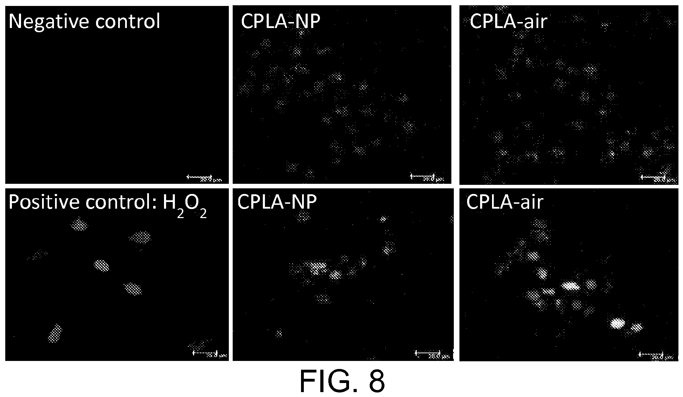

The photographs in Figure 8 indicate that before the photodynamic therapy, there is substantially no singlet oxygen in the sample (top panel). Also, for native CPLA photosensitizer particles, limited amount of singlet oxygen (green) is observed after the photodynamic therapy (bottom panel; CPLA-NP). In contrast, for cells treated with gas-loaded CPLA photosensitizer particles, abundant amount of singlet oxygen was generated after the photodynamic therapy (bottom panel; CPLA-air). These results suggest that the gas-loaded photosensitizer particles may result in more singlet oxygen generation, as compared with native photosensitizer particles.

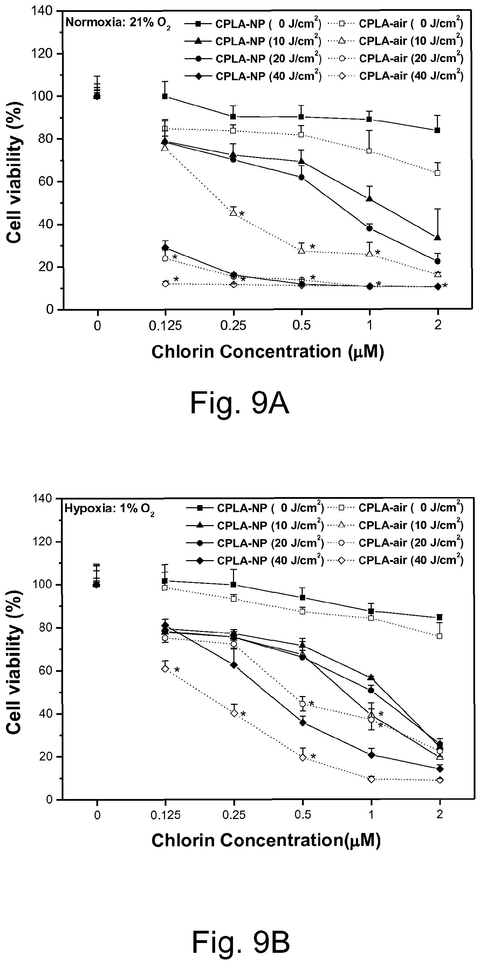

The phototoxicity mediated by the present gas-loaded photosensitizer particles was also investigated. Briefly, cells were cultured in medium containing various concentrations of native LN4 (CPLA-NP) or gas-loaded LA4 (CPLA-air) photosensitizer particles, under normoxia (21% O₂) or hypoxia (1% O₂) condition. After an incubation period of 24 hours, the cells were irradiated with light of different doses (661 nm) and then cells were incubated for another 24 hours under normoxia hypoxia condition. Thereafter, cells were washed using 1×PBS, followed by treatment with 5 mg/mL of MTT for 4 hours to allow the MTT to be metabolized. The culture media was then removed, and the cellular metabolic product, formazan, was resuspended in 100 µL DMSO with shaking for 30 minutes. The absorption of formazan was analyzed using microplate reader (SpectraMax M2^{e}, Molecular Devices). Results of each treatment group are summarized in Figure 9A and Figure 9B (* *p*<0.05).

Under normoxic environment, H1299 cells were more sensitive to photodynamic therapy, compared with those under hypoxic environment. For example, CPLA-NP and CPLA-air photosensitizer particles administered at a dose of 0.125 µM respectively resulted in a cell viability of about 30% and 10% in the normoxic group. By contrast, cell viabilities caused by the same photosensitizer particles under hypoxic condition were about 80% and 60%, respectively. This observation coincides with the fact that PDT is less effective in solid tumors experienced hypoxia.

On the other hand, for H1299 cells suffered from hypoxia, the gas-loaded CPLA photosensitizer particles (CPLA-air) exhibited better phototoxicity, compared with that of native CPLA photosensitizer particles (CPLA-NP). For example, hypoxic H1299 cells treated with 0.25 µM CPLA-NP and CPLA-air resulted in cell viability of about 65% and 40%, respectively. The increase in the phototoxicity of gas-loaded photosensitizer particles is believed to be associated with the increase in the production of singlet oxygen.

Taken together, results in this example indicate that (1) the present gas-loaded photosensitizer particles are capable of entering human cancerous cells; (2) the present gas-loaded photosensitizer particles, once inside the cancerous cells, result in morphological change of the cancerous cells upon appropriate light irradiation; (3) the present gas-loaded photosensitizer particles increase the production of singlet oxygen in cancerous cells; and (4) the present gas-loaded photosensitizer particles, under hypoxic conditions, are more toxic to cancerous cells than native photosensitizer particles are.

### Example 5

### Anti-tumor Effect of Gas-loaded Photosensitizer Particles

*In vivo* analysis was conducted to assess the anti-tumor effect of the present gas-loaded photosensitizer particles. Prior to injection of photosensitizer particles, all animals used in the present experimentation were housed in an animal room under temperature control (24-25°C) and 12:12 light-dark cycle. After the injection of photosensitizer particles, all mice (including the control group) were housed in a dark room. Standard laboratory chow and tap water were available *ad libitum.* The experiments procedures were approved by the National Science Council (Taipei City, Taiwan, R.O.C.) and were performed in compliance with national animal welfare regulations.

Human colon adenocarcinoma cells (HT29) cultured in high glucose DMEM medium (supplemented with 10% FBS and 1% antibiotics) were harvested at about 80% confluence.

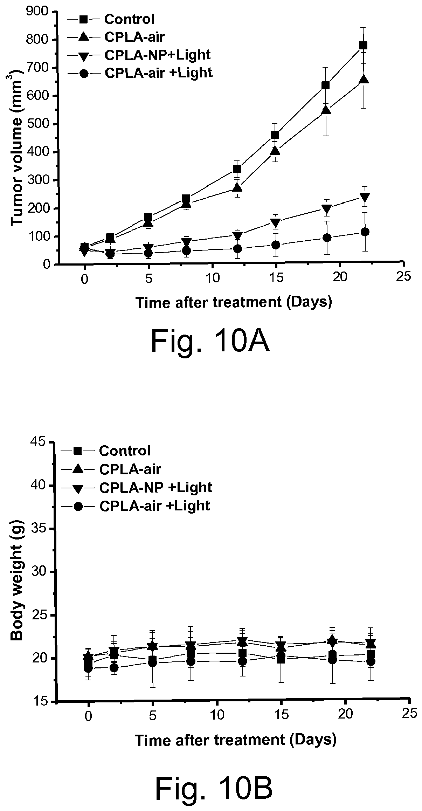

Nude mice (female; 6-8 weeks; 18-22 grams) were respectively inoculated with 2x10⁷ human colon adenocarcinoma cells (HT29 cells). Tumor size and volume were recorded for 31 days. Mice (5 mices pergroup) were injected with LA5 (the CPLA-air and the CPLA-air + light groups) or LN5 (CPLA-NP + light group) photosensitizer particles (0.6 mg/kg, single dose, i.v.) or double distilled H₂O (the control group), when tumor size reached about 50 mm³. 3 hours after the injection, mice in light-treated group were exposed to light (661 nm; 100 J/cm²), whereas mice in the CPLA-air and control groups received no irradiation. Mice were then monitored daily (for 3 weeks) for tumor size and body weight, and results are summarized in Figure 10A and Figure 10B.

The data in Figure 10A indicate that photodynamic therapy using the present gas-loaded photosensitizer particles significantly inhibited tumor growth, compared with the CPLA-air group in which no irradiation was given. Further, comparison between the CPLA-air + light and CPLA-NP + light groups reveals that the former is more effective in terms of antitumor activity. Also, at day 8 post-injection, all the animals survived, with no significant difference in body weight (Figure 10B).

Skin photosensitivity was also examined by visual observation of the photosensitizer particles administrated mice skin where the area was exposed to light (661 nm; 100 J/cm²) irradiation. Results indicate that no photosensitivity was elicited in mice treated with CPLA-NP + light irradiation or CPLA-air + light irradiation.

### Example 6

### Preparation and MRI Imaging of Mn-chelated Photosensitizer Particles

Mn-chelated CPCL (Mn-CPCL) photosensitizer particles were prepared using the water-in-oil solvent evaporation procedure. Briefly, Mn-CPCL conjugates prepared in Example 1 above were dissolved in acetone or THF and mix with polyethylene glycol 400 (0.1 mg). The solution was added into d.d. water drop by drop (drop rate: 0.25 mL/hr) with stirring and at room temperature to allow the self-assembly of photosensitizer particles. The solvent was then removed under vacuum. Summarized in Table 4 are the manufacturing parameters associated with the Mn-CPCL photosensitizer particles according to various embodiments of the present disclosure. The average hydrodynamic radius (R_{H}) and polydispersity (PDI) of the Mn-CPCL photosensitizer particles were measured using dynamic light scattering as described in Example 2, above, and the results are also summarized in Table 4.

**Table 4**

| **No** | Polymer (mg) | Solvent (mL) | d.d. H₂O (mL) | Stir (rpm) | R_{H} (nm) | PDI |
|---|---|---|---|---|---|---|
| **M1** | CPCL2, 2 | Acetone, 1 | 5 | 400 | 129.2±0.8386 | 0.087±0.009 |
| **M2** | CPCL4, 2 | THF, 2 | 5 | 450 | 130.7±0.5859 | 0.133±-0.011 |
| **M3** | CPCL4, 10 | THF, 5 | 30 | 600 | 97.20±0.5174 | 0115±0.017 |

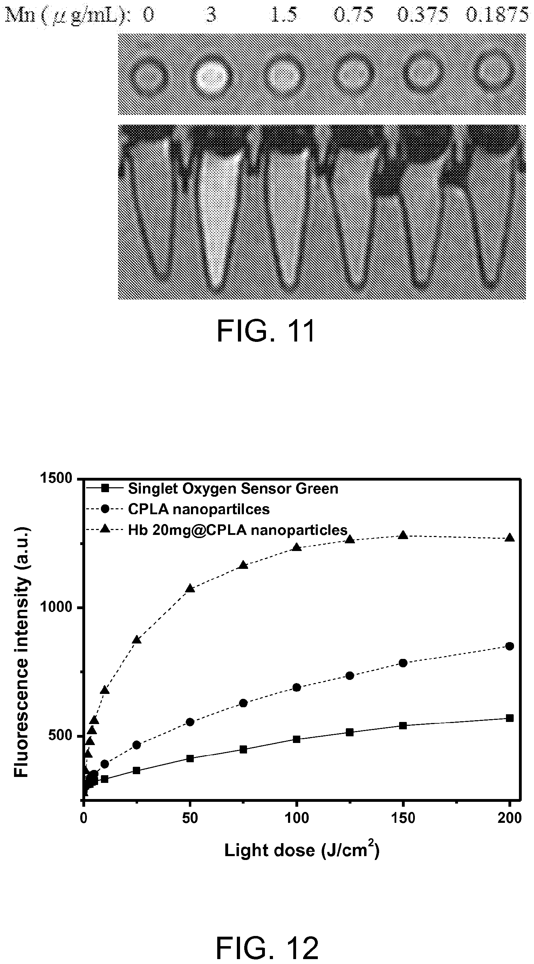

For MRI imaging, Mn-CPCL photosensitizer particles from Example M3 were dispersed in d.d. water to obtain Mn-CPCL photosensitizer particle solutions in the range of 0.1875-3 µg/mL. These solutions were than subjected to T1-weighted MRI with3 Tesla clinical MR imaging system (Signa Excite 3 T, GE Healthcare, USA). Images in Figure 11 are representative of samples of different concentrations, as well as the d.d. water (control). On a T1-weighted scan, the water content results in darker signals, and an MRI contrast agent that reduces the T1 relaxation time would give brighter signals. As is evident from Figure 11, the present Mn-CPCL photosensitizer particles exhibit a concentration-dependent MRI-enhancing effect. Image J free software was used to quantify the gray scale value of each sample, and results are summarized in Table 5.

**Table 5**

| **Mn (µg/mL)** | 3 | 1.5 | 0.75 | 0.375 | 0.1875 | 0 |
|---|---|---|---|---|---|---|
| **Sample** | 2602.428 | 2136.392 | 1851.506 | 1697.440 | 1642.229 | 1469.494 |
| **Background** | 1690.380 | 1672.482 | 1677.163 | 1700.120 | 1609.651 | 1648.970 |
| **Ratio (s/b)** | 1.539 | 1.277 | 1.103 | 0.998 | 1.020 | 0.891 |

The data in Table 5 indicate that the present Mn-CPCL photosensitizer particles effectively enhance the contrast of MRI imaging at a concentration of at least 1.5 µg/mL. Conventional MRI contrast agents, like conventional ultrasonic contrast agents, also experience drawbacks such as short blood circulation time. As discussed above, the present photosensitizer particles are advantageous in that they are more stable in blood circulation, and selective to tumor tissues, thereby making the present Mn-chelated photosensitizer particles ideal for use as MRI contrast agents.

### Example 7

### Preparation and Characterization of Hemoglobin-loaded CPLA Photosensitizer Particles

### Example 7.1: Preparation of Hemoglobin-loaded CPLA Photosensitizer Particles

Hemoglobin-loaded CPLA photosensitizer particles (hereinafter, Hb-loaded particles) were prepared under the same condition as described in Example 2.2, above, except that hemoglobin was added to the inner phase. Specifically, the CPLA3 conjugates prepared in Example 1.2, above was used as the starting conjugates, and the hemoglobin was dissolved in 100 µL of 0.1% (w/v) PVA aqueous solution in the amount specified in Table 6 below, and the hemoglobin-containing PVA solution was used as the inner water to form double emulsion (W/O/W) following the protocol set forth in Example 2.2. Physical properties of the Hb-loaded particles were measured as described above in Example 2.1, and the results are summarized in Table 6. Also included in Table 6 are the hemoglobin loading (Hb loading) and encapsulation efficiency of the Hb-loaded particles. To measure the Hb loading (load), the diluted sample was added to protein assay dye, read at 595 nm, and interpolated from a standard curve. The Encapsulation efficiency (E.E.) was calculated as the ratio of the actual Hb loading to the amount of hemoglobin used in the inner phase.

**Table 6**

| **No** | Hemoglobin in inner phase (mg) | R_{H} (d nm) | PDI | E.E. (%) | Load (mg) |
|---|---|---|---|---|---|
| **H1** | 5 | 1546±352.4 | 0.190±0.141 | 62.4 | 3.1 |
| **H2** | 10 | 359.4±7.304 | 0.341±0.027 | 57.1 | 5.7 |
| **H3** | 20 | 217.1±2.684 | 0.161±0.030 | 78.3 | 15.7 |
| **H4** | 25 | 329.2±8.000 | 0.174±0.020 | 72.6 | 18.1 |
| **H5** | 30 | 364.1±0.231 | 0.231±0.030 | 46.7 | 14.0 |
| **H6** | 40 | 712.4±22.07 | 0.573±0.040 | 68.7 | 27.5 |

The present Hb-loaded photosensitizer particles exhibited satisfactory drug load of at 60% in most experimental groups. The Hb-loaded photosensitizer particles with the highest drug load (that is, the H3 particles) were used in the subsequent experiments.

### Example 7.2: Singlet Oxygen Production Mediated by Native CPLA Photosensitizer Particles and Hemoglobin-loaded CPLA Photosensitizer Particles

To quantify the singlet oxygen (¹O₂) produced by native CPLA photosensitizer particles (LW4 particle from Example 2.2) and Hb-loaded CPLA photosensitizer particles (H3 particle from Example 7.1), Singlet Oxygen Sensor Green (Molecular Probes Inc., Leiden, the Netherlands) sensing probe was used. Briefly, 2 µL of 100 µM Singlet Oxygen Sensor Green (SOSG) was added into 198 µL of LW4 particle-containing or H3 particle-containing solution with a chlorin concentration of 2 µM. The sample was then exposed to light radiation (660 ± 10 nm) of various light doses to initiate the photochemical reaction. In the presence of singlet oxygen, the sensing probe emits a green fluorescence (Ex/Em = 488/525 nm) and the fluorescence intensity was determined using a SpectraMax M2^{e} multi-detection microplate reader and the results are provided in Figure 12.

As could be seen in Figure 12, both the CPLA photosensitizer particles (labeled as CPLA nanoparticles in the drawing) and the Hb-loaded CPLA photosensitizer particles (labeled as Hb 20 mg@CPLA nanoparticles in the drawing) significantly increase the production of the singlet oxygen, as is evidenced by the elevated fluorescence intensity as compared to the control, i.e., the Singlet Oxygen Sensor Green. Also, for both photosensitizer particles, the fluorescence intensity increases with the increases in light dose. It should be noted that under the same light dose, the fluorescence intensity produced by Hb-loaded particles is significantly higher than that of the native CPLA photosensitizer particles, indicating that the incorporation of hemoglobin in the photosensitizer particles increases the oxygen carrying capacity thereof.

### Example 7.3: Cytotoxicity and Phototoxicity of Native CPLA Photosensitizer Particles and Hemoglobin-loaded CPLA Photosensitizer Particles under Normoxia and Hypoxia Conditions

To investigate the therapeutic value of the present photosensitizer particles, cytotoxicity and phototoxicity of native CPLA photosensitizer particles (LW4 particle from Example 2.2) and Hb-loaded CPLA photosensitizer particles (H3 particle from Example 7.1) were assayed.

Briefly, H1299 cells were seeded onto 96-well plates at a density of 10,000 cells per 100 µL for each well and incubated with 3 mL of culture medium (10% FBS, supplemented with 1% antibiotics, 1mM sodium pyruvate and 2 mM L-glutamine) at 37°C for 24 hours under normoxia (21%) or hypoxia (1%) condition. Stock solutions of native CPLA photosensitizer particles (CPLA) or Hb-loaded particles (Hb@CPLA) were respectively added into the culture medium in two-fold serial dilution at the concentration ranging from 10 to 0.156 µM, and cells were incubated for a further 24 hours under normoxia or hypoxia condition. Thereafter, the cells were immediately exposed to light irradiation (0 and 10 J/cm²; 660 ± 10 nm at a total fluence rate of 19.5 mW/cm²).

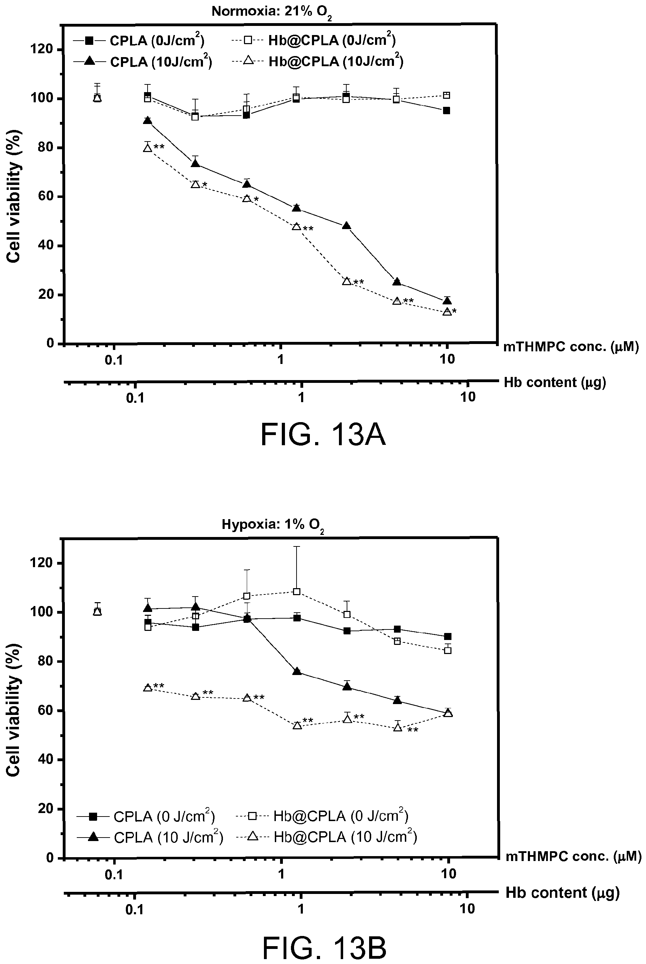

The *in vitro* cell viability was determinded 24 hours after the irradiation using the MTT assay as follows. The medium was replaced with 100 µL of medium containing MTT (2 mg/mL in PBS) and incubated for 2.5 hours, and then the medium was removed from the wells. Thereafter, 100 µL of DMSO was added under shaking for 30 minutes before the analysis. The presence of formazan (absorbence at 570 nm) was detected using microplate reader (SpectraMax M2^{e}, Molecular Devices). Results of each treatment group are summarized in Figure 13A and Figure 13B (* *p*<0.05 and ** *p*<0.005; compared to native CPLA photosensitizer particles subject to 10 J/cm² light irradiation).

Under both normoxic and hypoxic environments, both the native CPLA photosensitizing particles (labeled as CPLA (0J/cm²) in the drawing) and the Hb-loaded photosensitizing particles (labeled as Hb@CPLA (0J/cm²) in the drawing) exhibited limited or no cytotoxicity. Specifically, referring to Figure 13 A, when the cells were subject to light of 0 J/cm² under normoxia condition, the cell viability is greater than 90% in all treatment groups. Similar trend is seen Figure 13B. Specifically, for cells subject to light of 0 J/cm² under hypoxia condition, only one treatment group (10 µM Hb@CPLA) gave rise to a cell viability slightly lower than 90%.

By comparing the phototoxicities of native CPLA photosensitizing particles (labeled as CPLA (10J/cm²) in the drawing) under the normoxic and hypoxic conditions, it is found that H1299 cells were less sensitive to photodynamic therapy under hypoxic environment. For example, native CPLA photosensitizer particles administered at doses of 1.25 µM and 5 µM respectively resulted in a cell viability of about 55% and 25% in the normoxic group. By contrast, cell viabilities caused by the same photosensitizer particles under hypoxic condition were about 75% and 65%, respectively. This observation is in line with the results in Example 4, above and coincides with the fact that PDT is less effective in solid tumors suffering from hypoxia.

The use of Hb-loaded photosensitizing particles (labeled as Hb@CPLA (10J/cm²) in the drawing) counteracts the adverse effect of the hypoxic environment on the photodynamic therapy. Referring to Figure 13B, at most concentrations, the Hb-loaded photosensitizing particles exhibited greater phototoxicity compared to the corresponding native CPLA photosensitizing particles; the only exception is the group treated with 10 µM Hb-loaded photosensitizing particles, in which the cell viability was substantially the same as that of the group treated with 10 µM native CPLA photosensitizing particles. The Hb-loaded photosensitizing particles are also more effective PDT agents under the normoxic condition. For example, in Figure 13A, at all the tested concentrations, the Hb-loaded photosensitizing particles resulted in a lower cell viability than the corresponding native CPLA photosensitizing particles did, and the difference between each paired treatment group is statistically significant. The increase in the phototoxicity of Hb-loaded photosensitizer particles is believed to be associated with the increase in the production of singlet oxygen resulting from the enhanced oxygen delivery capacity of Hb-loaded photosensitizer particles.

In sum, the results in this working example indicate that the present Hb-loaded photosensitizer particles, like the native CPLA photosensitizer particles, can enter human cancerous cells, and result in cell death of the cancerous cells upon appropriate light irradiation. Also, the present Hb-loaded photosensitizer particles increase the production of singlet oxygen in cancerous cells, and hence they are more effective PDT agents than native CPLA photosensitizer particles, under both normoxic and hypoxic conditions.

### Example 8

### Preparation and Characterization of Perfluorodecalin-loaded CPLA Photosensitizer Particles

### Example 8.1: Preparation of Perfluorodecalin-loaded CPLA Photosensitizer Particles

Native CPLA photosensitizer particles and perfluorodecalin-loaded CPLA photosensitizer particles (hereinafter, the PFD-loaded photosensitizer particles) were prepared by the solvent evaporation emulsion. Briefly, 10 or 15 mg of CPLA4 conjugates from example 1.2, above, was dissolved in 200 µL of dichloromethane and 400 µL of diethyl ether with or without the addition of 50 or 75 µL of PFD; the mixture was subsequently emulsified with 5.0 mL of 0.5 % (w/v) Pluronic F-68 aqueous solution with a sonicator (20 kHz) for 5 minutes. Thereafter, the emulsion solution was stirred for 24 hours to allow the formation of CPLA photosensitizer particles by slow evaporation. To collect the as-formed particles, excess Pluronic F-68 was removed by centrifugation at 7,000 g for 60 minutes, and the sediment was then washed by double-distilled water for three times. The average hydrodynamic radius (R_{H}) and polydispersity (PDI) of the PFD-loaded particles were measured as described above in Example 2.1; the PFD loading of the PFD-loaded particles was determined using 19F NMR spectroscopy with sodium fluoride as an internal standard; and the encapsulation efficiency was determined using the equation described above in Example 7.1. Results are summarized in Table 7.

**Table 7**

| No. | CPLA4 (mg) | PFD (µL) | R_{H}(d.nm) | PDI | E.E. (%) |
|---|---|---|---|---|---|
| P1 | 10 | 75 | 272.3± 7.894* | 0.400± 0.021* | N/A |
| P2 | 10 | 50 | 257.0± 7.565 | 0.303± 0.017 | 69 |
| P3 | 15 | 50 | 307.1± 2.108 | 0.200± 0.020 | N/A |

| | | | | | |
|---|---|---|---|---|---|
| * Sample without purification. | | | | | |

The P2 particles having an encapsulation efficiency of 69% were used in the subsequent experiments.

### Example 8.2: Singlet Oxygen Production Mediated by Native CPLA Photosensitizer Particles and PFD-loaded CPLA Photosensitizer Particles

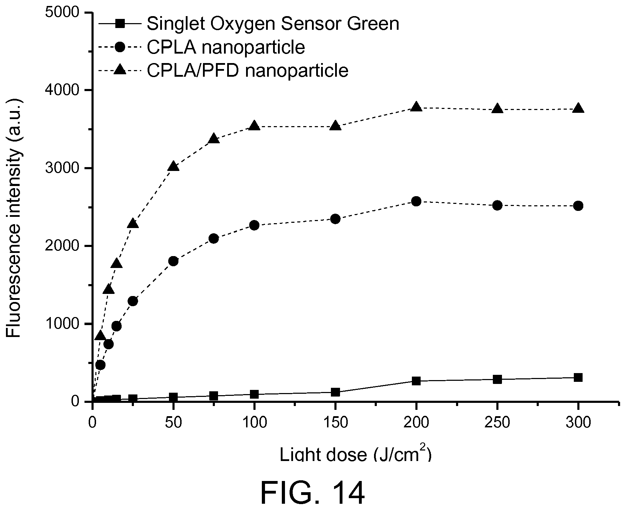

Evaluation of the amount of singlet oxygen produced by native CPLA photosensitizer particles and P2 PFD-loaded CPLA photosensitizer particles, both from Example 8.1, above, was performed by mixing 2 µL of 100 µM SOSG and 198 µL of native CPLA particle-containing or P2 particle-containing solution (mTHMPC concentration: 2 µM), and then irradiating the mixture with light (660 ± 10 nm) of various light doses. The singlet oxygen was quantified by method set forth in Example 7.2, above, and the results are provided in Figure 14.

In Figure 14, it can be seen that the production of singlet oxygen follows a similar trend to that observed in Figure 12 from Example 7.2, above. Specifically, both the native CPLA photosensitizer particles (labeled as CPLA nanoparticle in the drawing) and the PFD-loaded CPLA photosensitizer particles (labeled as CPLA/PFD nanoparticle in the drawing) significantly increase the production of the singlet oxygen after light irradiation, with the latter mediated a greater amount of singlet oxygen production. These data indicate that the incorporation of perfluorocarbon such as perfluorodecalin in the photosensitizer particles significantly increases the oxygen carrying capacity thereof.

### Example 8.3: Cytotoxicity and Phototoxicity of Native CPLA Photosensitizer Particles and PFD-loaded CPLA Photosensitizer Particles under Normoxia and Hypoxia Conditions

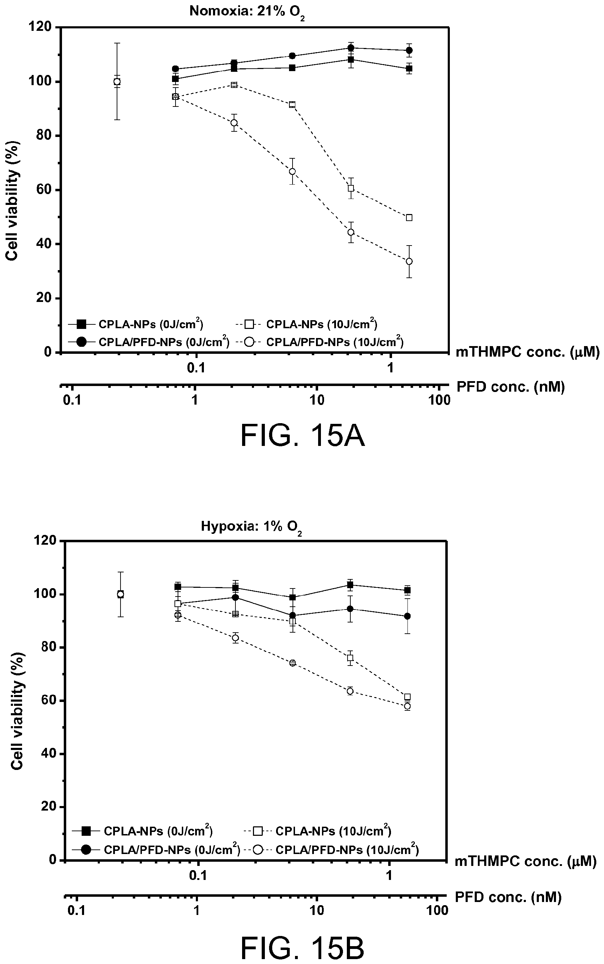

The cytotoxicity and phototoxicity of native CPLA photosensitizer particles and P2 PFD-loaded CPLA photosensitizer particles, both from Example 8.1, above, were assayed using the protocol set forth in Example 7.3, above, except that the culture medium contained 1.25 to 0.0786 µM photosensitizer particles in two-fold serial dilution. Results of cell viability under normoxia and hypoxia are provided in Figure 15A and Figure 15B, respectively.

In Figure 15A, it is observed that both the native CPLA photosensitizing particles (labeled as CPLA-NPs (0J/cm²) in the drawing) and the PFD-loaded photosensitizing particles (labeled as CPLA/PFD-NPs (0J/cm²) in the drawing) elicited almost no cytotoxicity under normoxic condition. Similar results can be found in Figure 15B, in which only limited cytotoxicity was found under hypoxic condition.

On the other hand, both the native CPLA photosensitizing particles (labeled as CPLA-NPs (10J/cm²) in the drawing) and the PFD-loaded photosensitizing particles (labeled as CPLA/PFD-NPs (10J/cm²) in the drawing) exhibited notable phototoxicity under both normoxia and hypoxia. For example, native CPLA photosensitizer particles and PFD-loaded photosensitizing particles administered at doses of 0.625 respectively resulted in a cell viability of about 60% and 45% in the normoxic group and about 75% and 65% in the hypoxic group. These results indicate that the PFD-loaded photosensitizing particles, with their increased oxygen content, enhance the efficacy of photodynamic therapy under hypoxic conditions.

In conclusion, the results in this working example indicate that the present PFD-loaded photosensitizer particles can also enter human cancerous cells and elicit phototoxicity therein upon appropriate light irradiation. Further, due to the enhanced oxygen load of the PFD-loaded photosensitizer particles, they are more effective PDT agents than native CPLA photosensitizer particles.

### Example 9

### Anti-tumor Effect of Native CPLA Photosensitizer particles and Gas-loaded CPLA Photosensitizer Particles in Hypoxia Tumor Model

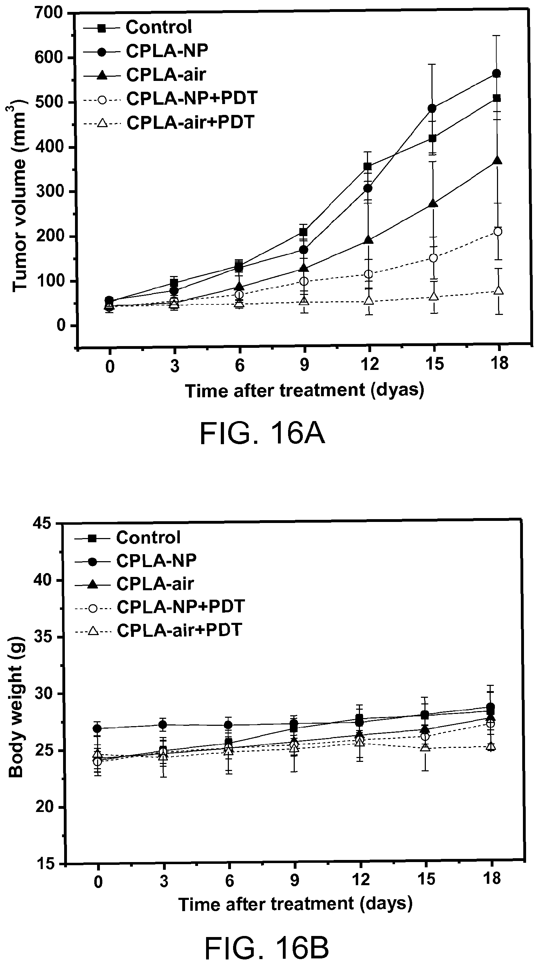

NOD-SCID mice (female; 6-8 weeks; 20 grams) were respectively inoculated with 1x10⁷ human non-small cell lung carcinoma cells (H1299 cells). Tumor size and volume were recorded for 18 days. Mice (5 mice/group) were injected with 1.0 mg/kg (single dose, i.v.) native CPLA photosensitizer particles (LN5 particles from Example 2.1, above) or gas-loaded CPLA photosensitizer particles (LA5 particles or from Example 2.1) or double distilled H₂O (the control group), when tumor size reached about 50 mm³. Three hours after the injection, mice in PDT group were exposed to light (661 nm; 100 J/cm²), whereas mice in the CPLA-NP, CPLA-air and control groups received no irradiation. Mice were then monitored daily (for 3 weeks) for tumor size and body weight, and results are depicted in Figure 16A and Figure 16B, respectively.

The results in Figure 16A indicate that photodynamic therapy using the present native CPLA photosensitizer particles (labeled as CPLA-NP+PDT in the drawing) and gas-loaded CPLA photosensitizer particles (labeled as CPLA-air+PDT in the drawing) significantly inhibited tumor growth, compared with the control group, the CPLA-NP group and CPLA-air group in which no irradiation was given. Further, the reduced tumor size observed in mice treated with gas-loaded CPLA photosensitizer particles, as compared with native CPLA photosensitizer particles, suggests that gas-loaded CPLA photosensitizer particles were more effective PDT agents in treating solid tumor that often experiencing hypoxia. Also, there was no significant difference in body weight among all groups (Figure 16B).

### Example 10

### Hemolytic Activity of Native CPLA Photosensitizer Particles

Blood samples were collected from the heart of ICR strain mice using blood collection tubes containing anticoagulant (BD Vacutainer) and mixed well. The samples were centrifuged at 1,500 g for 5 minutes, and then washed with equal amount of PBS. The centrifuging and washing steps were repeated for 6 times. Native CPLA photosensitizing particles (LN1 particles from Example 2.1, above) were respectively added into the samples medium in two-fold serial dilution at the chlorin concentration ranging from 0.03 to 2 µM; double distilled water and PBS were respectively added for positive and negative controls. Thereafter, the samples were bathed in water at 37°C for 30 or 60 minutes and then centrifuged at 1,500 g for 5 minutes. The supernatant was transferred to a 96-well plate, and the light absorbance at 405 nm was read using ELISA Reader.

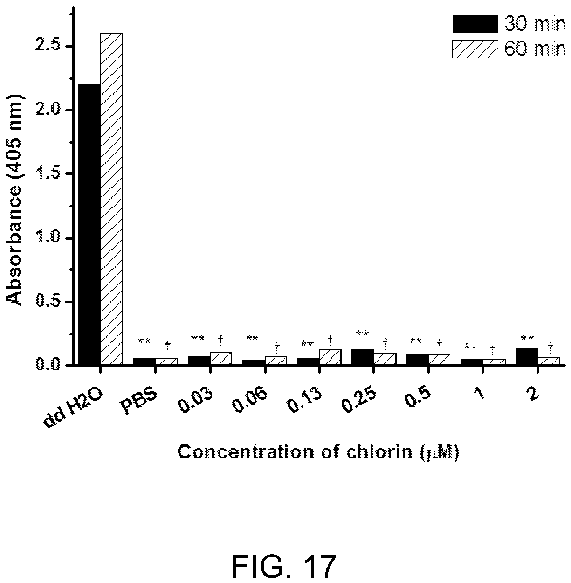

The results of hemolytic analysis are summarized in Figure 17 (**p<0.05and ^{†}p<0.05; compared to the positive control after 30 and 60 minutes treatment, respectively). In Figure 17, hemolysis was observed with blood samples treated with double distilled water, while no hemolysis was found in PBS-treated samples. On the other hand, none of the blood samples treated with present native CPLA photosensitizer particles exhibited significant hemolytic activity. Notably, the native CPLA photosensitizer particles, even administered at high concentrations such as 1 µM and 2 µM, did not elicited hemolysis.

### Example 11

### In Vivo Photohypersensitivity of Mice Skin to Native CPLA Photosensitizer Particles and Gas-loaded CPLA Photosensitizer Particles

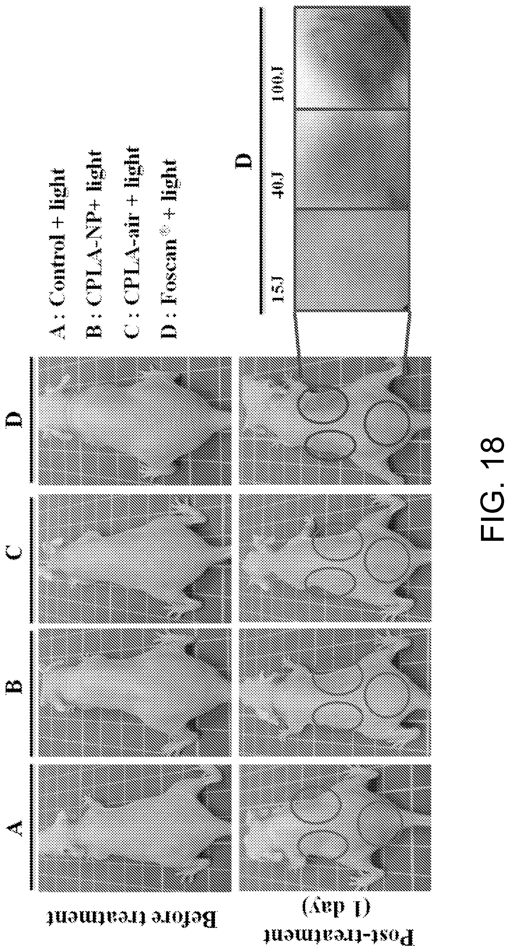

It is well known that some PDT agents result in photohypersensitivity of the skin, which causes significant inconvenience in the daily life of the patient. Typical signs of photohypersensitivity include redness and swelling of the skin, visible angiopathy along with the formation of skin debris and even eschar in server cases. To investigate whether the present photosensitizer particles may elicit photohypersensitivity in the patient, 0.3 mg/kg native CPLA photosensitizer particles (LN1 particles from Example 2.1, above) and 0.3 mg/kg gas-loaded CPLA photosensitizer particles (LA1 particles from Example 2.1, above) were respectively injected to the tail of nude mice via intravenous injection 3 hours prior to light irradiation (15, 40 or 100 J/cm²). Foscan® (0.3 mg/kg) was used as the positive control, while 1xPBS (100 µL) was used as negative control. Photographs of the treatment sites taken before and after the photodynamic therapy are provided in Figure 18.

In the positive control group (labeled as group D in the drawing), mice treated with Foscan® and 15 or 40 J/cm² of light dose experienced little to mild photohypersensitivity, judging from the red and light purple plaques on the skin. On the other hand, the dark red and purple plaque on the mice skin indicates that significant photohypersensitivity occurred in mice treated with Foscan® at a higher light dose of 100 J/cm². These data suggest that the extent of the photohypersensitivity aggravates with the increase of light dose.

In comparison, no sign of photosensitivity was observed in mice treated with native CPLA photosensitizer particles (labeled as group B in the drawing) or gas-loaded CPLA photosensitizer particles (labeled as group C in the drawing) at all the give light doses. The same goes to mice in the negative control group (labeled as group A in the drawing).

In view of the foregoing, the present native and gas-loaded CPLA photosensitizer particles do not elicit photohypersensitivity in mice under the specified concentrations and light doses.

## Claims

1. A photosensitizer particle, comprising,
a shell consisting essentially of a plurality of photosensitizer conjugates, wherein each of the plurality of photosensitizer conjugates consists of (1) a photosensitizer, and (2) one to four biodegradable polymers covalently bound to the photosensitizer; and
a core, filled with gas, liquid or a mixture of gas and liquid.

2. The photosensitizer particle of claim 1, wherein the core is filled with gas or a mixture of gas and liquid, and the gas is air, oxygen, a fluorocarbon gas, or a combination thereof.

3. The photosensitizer particle of claim 1, wherein the photosensitizer is porphyrin, chlorin, phthalocyanine, bacteriochlorin, or methylene blue.

4. The photosensitizer particle of claim 1, wherein the biodegradable polymer is polylactic acid, polycaprolactone, or polyglycolic acid.

5. The photosensitizer particle of claim 1, wherein the shell has an aggregation number of 2,000-15,000.

6. The photosensitizer particle of claim 1, wherein the photosensitizer particle has a polydispersity index of 0.05-0.3.

7. The photosensitizer particle of claim 1, wherein the photosensitizer is *meta*-tetra-3-hydroxymethyl phenyl chlorin (m-THPMPC) and the biodegradable polymer is polylactic acid.

8. The photosensitizer particle of claim 1, wherein the core is filled with gas, and the photosensitizer particle is prepared by:
(a) dissolving the photosensitizer conjugates in an organic solvent to produce a conjugate solution, wherein the organic solvent is acetone or tetrahydrofuran; and
(b) drop-wisely adding the conjugate solution into deionized water with stirring while pumping gas into the mixture.

9. The photosensitizer particle of claim 8, wherein,
the ratio of the photosensitizer conjugates to the organic solvent is 1:10 to 1:100 (m/v);
the volume ratio of the organic solvent to the deionized water is 1:1 to 1:20; and
the pumping is continued until at least 30 minutes after the depletion of the conjugate solution.

10. The photosensitizer particle of claim 1, wherein the core is filled with gas, and the photosensitizer particle is prepared by:
(a) dissolving the photosensitizer conjugates in dichloromethane to obtain a conjugate solution;
(b) adding a first polyvinyl alcohol (PVA) aqueous solution into the conjugate solution with sonication to produce a water-in-oil emulsion;
(c) adding the water-in-oil emulsion into a second PVA solution with sonication to produce a water-in-oil-in-water emulsion;
(d) removing the dichloromethane from the water-in-oil-in-water emulsion; and
(e) removing the PVA to produce the photosensitizer particle.

11. The photosensitizer particle of claim 10, wherein
the ratio of the photosensitizer conjugates to the dichloromethane is 1:1 to 100:1 (m/v);
the concentration of the first PVA solution is 0.1 to 10% (w/v);
the volume ratio of the dichloromethane to the first PVA aqueous solution is 1:1 to 100:1;
the concentration of the second PVA solution is 0.1 to 10% (w/v); and
the volume ratio of the dichloromethane to the second PVA aqueous solution is 1:1 to 1:10

12. The photosensitizer particle of claim 1, wherein the photosensitizer of at least one of the plurality of photosensitizer conjugates comprises a magnetic contrast-enhancing material,

13. The photosensitizer particle of claim 1, wherein the liquid contains at least one oxygen carrier.

14. Use of a photosensitizer particle for the manufacture of a medicament for use in a photodynamic therapy, wherein the photosensitizer particle is the photosensitizer particle of any of claims 1 to 13.

## Patentansprüche

1. Photosensibilisatorpartikel, umfassend
eine Hülle, die sich im Wesentlichen aus einer Vielzahl von Photosensibilisatorkonjugaten zusammensetzt, wobei jedes der Vielzahl von Photosensibilisatorkonjugaten aus (1) einem Photosensibilisator und (2) ein bis vier biologisch abbaubaren Polymeren besteht, die kovalent an den Photosensibilisator gebunden sind, und
einen Kern, der mit Gas, einer Flüssigkeit oder einem Gemisch aus Gas und einer Flüssigkeit gefüllt ist.

2. Photosensibilisatorpartikel nach Anspruch 1, bei welchem der Kern mit Gas oder einem Gemisch aus Gas und einer Flüssigkeit gefüllt ist, wobei das Gas Luft, Sauerstoff, ein Fluorkohlenstoffgas oder eine Kombination davon ist.

3. Photosensibilisatorpartikel nach Anspruch 1, bei welchem der Photosensibilisator Porphyrin, Chlorin, Phthalocyanin, Bakteriochlorin oder Methylenblau ist.

4. Photosensibilisatorpartikel nach Anspruch 1, bei welchem das biologisch abbaubare Polymer Polymilchsäure, Polycaprolacton oder Polyglykolsäure ist.

5. Photosensibilisatorpartikel nach Anspruch 1, bei welchem die Hülle eine Aggregationszahl von 2000 - 15000 hat.

6. Photosensibilisatorpartikel nach Anspruch 1, bei welchem das Photosensibilisatorpartikel einen Polydispersitätsindex von 0,05 - 0,3 aufweist.

7. Photosensibilisatorpartikel nach Anspruch 1, bei welchem der Photosensibilisator *Meta*-tetra-3-hydroxymethylphenylchlorin (m-THPMPC) ist und das biologisch abbaubare Polymer Polymilchsäure ist.

8. Photosensibilisatorpartikel nach Anspruch 1, bei welchem der Kern mit Gas gefüllt ist, wobei das Photosensibilisatorpartikel hergestellt ist durch:
(a) Lösen der Photosensibilisatorkonjugate in einem organischen Lösungsmittel zur Erzeugung einer Konjugatlösung, wobei das organische Lösungsmittel Aceton oder Tetrahydrofuran ist, und
(b) tropfenweise Zugabe der Konjugatlösung in deionisiertes Wasser unter Rühren während Gas in die Mischung gepumpt wird.

9. Photosensibilisatorpartikel nach Anspruch 8, bei welchem
das Verhältnis von Photosensibilisatorkonjugaten zu dem organischen Lösungsmittel 1:10 bis 1:100 (m/v) beträgt,
das Volumenverhältnis von dem organischen Lösungsmittel zu deionisiertem Wasser 1:1 bis 1:20 beträgt und
das Pumpen nach dem Aufbrauchen der Konjugatlösung bis mindestens 30 Minuten fortgesetzt wird.

10. Photosensibilisatorpartikel nach Anspruch 1, bei welchem der Kern mit Gas gefüllt ist, wobei das Photosensibilisatorpartikel hergestellt ist durch:
(a) Lösen der Photosensibilisatorkonjugate in Dichlormethan, um eine Konjugatlösung zu erhalten,
(b) Zugabe einer ersten wässrigen Polyvinylalkohol (PVA) - Lösung in die Konjugatlösung unter Ultraschall, um eine Wasser-in-Öl-Emulsion zu erzeugen,
(c) Zugabe der Wasser-in-Öl-Emulsion in eine zweite PVA-Lösung unter Ultraschall, um eine Wasser-in-Öl-in-Wasser-Emulsion zu erzeugen,
(d) Entfernen des Dichlormethans aus der Wasser-in-Öl-in-Wasser- Emulsion und
(e) Entfernen des PVA, um das Photosensibilisatorpartikel zu erzeugen.

11. Photosensibilisatorpartikel nach Anspruch 10, bei welchem
das Verhältnis von Photosensibilisatorkonjugaten zu Dichlormethan 1:1 bis 100:1 (m/v) beträgt,
die Konzentration der ersten PVA-Lösung 0,1 bis 10 % (w/v) beträgt,
das Volumenverhältnis von Dichlormethan zu der ersten wässrigen PVA - Lösung 1:1 bis 100:1 beträgt,
die Konzentration der zweiten PVA-Lösung 0,1 bis 10 % (w/v) beträgt und
das Volumenverhältnis von Dichlormethan zu der zweiten wässrigen PVA - Lösung 1:1 bis 1:10 beträgt.

12. Photosensibilisatorpartikel nach Anspruch 1, bei welchem der Photosensibilisator von wenigstens einem der Vielzahl von Photosensibilisatorkonjugaten ein magnetisches kontrastverstärkendes Material umfasst.

13. Photosensibilisatorpartikel nach Anspruch 1, bei welchem die Flüssigkeit wenigstens einen Sauerstoffträger beinhaltet.

14. Verwendung eines Photosensibilisatorpartikels zur Herstellung eines in einer photodynamischen Therapie verwendbaren Medikaments, wobei das Photosensibilisatorpartikel ein Photosensibilisatorpartikel nach einem der Ansprüche 1 bis 13 ist.

## Revendications

1. Particule de photosensibilisateur, comprenant,
une écorce consistant essentiellement en une pluralité de conjugués de photosensibilisateur, dans lesquels chacun de la pluralité de conjugués de photosensibilisateur consiste en (1) un photosensibilisateur et (2) un à quatre polymères biodégradables liés en covalence au photosensibilisateur; et
un noyau, rempli de gaz, de liquide ou d'un mélange de gaz et de liquide.

2. Particule de photosensibilisateur selon la revendication 1, dans laquelle le noyau est rempli de gaz ou d'un mélange de gaz et de liquide, et le gaz est de l'air, de l'oxygène, un gaz de fluorocarbone, ou une combinaison de ceux-ci.

3. Particule de photosensibilisateur selon la revendication 1, dans laquelle le photosensibilisateur est de la porphyrine, du chlore, de la phtalocyanine, de la bactériochlorine, ou du bleu de méthylène.

4. Particule de photosensibilisateur selon la revendication 1, dans laquelle le polymère biodégradable est de l'acide polylactique, de la polycaprolactone, ou de l'acide polyglycolique.

5. Particule de photosensibilisateur selon la revendication 1, dans laquelle l'écorce a un nombre d'agrégation de 2 000 à 15 000.

6. Particule de photosensibilisateur selon la revendication 1, dans laquelle la particule de photosensibilisateur a un indice de polydispersité de 0,05 à 0,3.

7. Particule de photosensibilisateur selon la revendication 1, dans laquelle le photosensibilisateur est du méta-tétra-3-hydroxyméthyle phényle chlore (m-THPMPC) et le polymère biodégradable est de l'acide polylactique.

8. Particule de photosensibilisateur selon la revendication 1, dans laquelle le noyau est rempli de gaz et la particule de photosensibilisateur est préparée en:
(a) dissolvant les conjugués de photosensibilisateur dans un solvant organique pour produire une solution conjuguée, dans laquelle le solvant organique est de l'acétone ou du tétrahydrofuranne; et
(b) ajoutant au goutte-à-goutte la solution conjuguée dans de l'eau déionisée avec agitation tout en pompant du gaz dans le mélange.

9. Particule de photosensibilisateur selon la revendication 8, dans laquelle,
le rapport des conjugués de photosensibilisateur sur le solvant organique est de 1:10 à 1:100 (M/V);
le rapport volumique du solvant organique sur l'eau déionisée est de 1:1 à 1:20; et
le pompage est continué jusqu'à au moins 30 minutes après l'épuisement de la solution conjuguée.

10. Particule de photosensibilisateur selon la revendication 1, dans laquelle le noyau est rempli de gaz et la particule de photosensibilisateur est préparée en:
(a) dissolvant les conjugués de photosensibilisateur dans du dichlorométhane pour obtenir une solution conjuguée;
(b) ajoutant une première solution aqueuse d'alcool de polyvinyle (PVA) dans la solution conjuguée avec sonification pour produire une émulsion eau dans l'huile;
(c) ajoutant l'émulsion eau dans l'huile dans une seconde solution de PVA avec sonification pour produire une émulsion eau dans l'huile dans l'eau;
(d) enlevant le dichlorométhane de l'émulsion eau dans l'huile dans l'eau; et
(e) enlevant le PVA pour produire la particule de photosensibilisateur.

11. Particule de photosensibilisateur selon la revendication 10, dans laquelle
le rapport des conjugués de photosensibilisateur sur le dichlorométhane est de 1:1 à 100:1 (M/V);
la concentration de la première solution de PVA est de 0,1 à 10 % (W/V);
le rapport volumique du dichlorométhane sur la première solution aqueuse de PVA est 1:1 à 100:1;
la concentration de la seconde solution de PVA est de 0,1 à 10 % (W/V); et
le rapport volumique du dichlorométhane sur la seconde solution aqueuse de PVA est 1:1 à 1:10.

12. Particule de photosensibilisateur selon la revendication 1, dans laquelle le photosensibilisateur d'au moins un de la pluralité de conjugués de photosensibilisateur comprend une matière magnétique améliorant le contraste.

13. Particule de photosensibilisateur selon la revendication 1, dans laquelle le liquide contient au moins un porteur d'oxygène.

14. Utilisation d'une particule de photosensibilisateur pour la fabrication d'un médicament à utiliser dans une thérapie photodynamique, dans laquelle la particule de photosensibilisateur est la particule de photosensibilisateur selon l'une quelconque des revendications 1 à 13.
